(19) Europäisches Patentamt
European Patent Office
Office européen des brevets

(11) EP 3 858 377 A1

(12) # EUROPEAN PATENT APPLICATION

published in accordance with Art. 153(4) EPC

(43) Date of publication:
**04.08.2021 Bulletin 2021/31**

(21) Application number: **19864323.1**

(22) Date of filing: **27.09.2019**

(51) Int Cl.:
***A61K 39/00*** *(2006.01)* ***A61K 38/10*** *(2006.01)*
***A61K 38/16*** *(2006.01)* ***A61K 47/69*** *(2017.01)*
***A61P 35/00*** *(2006.01)* ***C07K 14/82*** *(2006.01)*
***C07K 19/00*** *(2006.01)*

(86) International application number:
**PCT/JP2019/038223**

(87) International publication number:
**WO 2020/067453 (02.04.2020 Gazette 2020/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.09.2018 JP 2018185532**

(71) Applicant: **Sumitomo Dainippon Pharma Co., Ltd.**
**Chuo-ku**
**Osaka-shi**
**Osaka 541-8524 (JP)**

(72) Inventors:
- **MORITA, Akihiro**
  **Suita-shi, Osaka 564-0053 (JP)**
- **TSUZUKU, Takuma**
  **Tokyo 104-8356 (JP)**
- **YOSHINAGA, Yuko**
  **Suita-shi, Osaka 564-0053 (JP)**

(74) Representative: **Vossius & Partner**
**Patentanwälte Rechtsanwälte mbB**
**Siebertstraße 3**
**81675 München (DE)**

(54) **INJECTABLE COMPOSITION**

(57) The present invention relates to a lyophilized preparation comprising two or more cancer antigen peptides derived from WT1 protein with an activity of inducing cytotoxic T lymphocytes for cancer peptide vaccine therapy.

EP 3 858 377 A1

SEQUENCE LISTING

<110> Sumitomo Dainippon Pharma Co., Ltd.

<120> Composition for injection

<130> 675165

<150> JP 2018-185532
<151> 2018-09-28

<160> 40

<170> PatentIn version 3.5

<210> 1
<211> 449
<212> PRT
<213> Homo sapiens

<400> 1

```
Met Gly Ser Asp Val Arg Asp Leu Asn Ala Leu Leu Pro Ala Val Pro
1               5                   10                  15

Ser Leu Gly Gly Gly Gly Gly Cys Ala Leu Pro Val Ser Gly Ala Ala
            20                  25                  30

Gln Trp Ala Pro Val Leu Asp Phe Ala Pro Pro Gly Ala Ser Ala Tyr
        35                  40                  45

Gly Ser Leu Gly Gly Pro Ala Pro Pro Pro Ala Pro Pro Pro Pro Pro
    50                  55                  60

Pro Pro Pro Pro His Ser Phe Ile Lys Gln Glu Pro Ser Trp Gly Gly
65                  70                  75                  80

Ala Glu Pro His Glu Glu Gln Cys Leu Ser Ala Phe Thr Val His Phe
                85                  90                  95

Ser Gly Gln Phe Thr Gly Thr Ala Gly Ala Cys Arg Tyr Gly Pro Phe
            100                 105                 110

Gly Pro Pro Pro Pro Ser Gln Ala Ser Ser Gly Gln Ala Arg Met Phe
        115                 120                 125

Pro Asn Ala Pro Tyr Leu Pro Ser Cys Leu Glu Ser Gln Pro Ala Ile
    130                 135                 140

Arg Asn Gln Gly Tyr Ser Thr Val Thr Phe Asp Gly Thr Pro Ser Tyr
145                 150                 155                 160

Gly His Thr Pro Ser His His Ala Ala Gln Phe Pro Asn His Ser Phe
```

165 170 175

Lys His Glu Asp Pro Met Gly Gln Gln Gly Ser Leu Gly Glu Gln Gln
180 185 190

Tyr Ser Val Pro Pro Pro Val Tyr Gly Cys His Thr Pro Thr Asp Ser
195 200 205

Cys Thr Gly Ser Gln Ala Leu Leu Leu Arg Thr Pro Tyr Ser Ser Asp
210 215 220

Asn Leu Tyr Gln Met Thr Ser Gln Leu Glu Cys Met Thr Trp Asn Gln
225 230 235 240

Met Asn Leu Gly Ala Thr Leu Lys Gly Val Ala Ala Gly Ser Ser Ser
245 250 255

Ser Val Lys Trp Thr Glu Gly Gln Ser Asn His Ser Thr Gly Tyr Glu
260 265 270

Ser Asp Asn His Thr Thr Pro Ile Leu Cys Gly Ala Gln Tyr Arg Ile
275 280 285

His Thr His Gly Val Phe Arg Gly Ile Gln Asp Val Arg Arg Val Pro
290 295 300

Gly Val Ala Pro Thr Leu Val Arg Ser Ala Ser Glu Thr Ser Glu Lys
305 310 315 320

Arg Pro Phe Met Cys Ala Tyr Pro Gly Cys Asn Lys Arg Tyr Phe Lys
325 330 335

Leu Ser His Leu Gln Met His Ser Arg Lys His Thr Gly Glu Lys Pro
340 345 350

Tyr Gln Cys Asp Phe Lys Asp Cys Glu Arg Arg Phe Ser Arg Ser Asp
355 360 365

Gln Leu Lys Arg His Gln Arg Arg His Thr Gly Val Lys Pro Phe Gln
370 375 380

Cys Lys Thr Cys Gln Arg Lys Phe Ser Arg Ser Asp His Leu Lys Thr
385 390 395 400

His Thr Arg Thr His Thr Gly Lys Thr Ser Glu Lys Pro Phe Ser Cys
405 410 415

```
Arg Trp Pro Ser Cys Gln Lys Lys Phe Ala Arg Ser Asp Glu Leu Val
            420                 425                 430

Arg His His Asn Met His Gln Arg Asn Met Thr Lys Leu Gln Leu Ala
        435                 440                 445

Leu
```

<210> 2
<211> 9
<212> PRT
<213> Artificial Sequence

<220>
<223> Peptide

<400> 2

```
Arg Met Phe Pro Asn Ala Pro Tyr Leu
1               5
```

<210> 3
<211> 9
<212> PRT
<213> Artificial Sequence

<220>
<223> Peptide

<400> 3

```
Cys Met Thr Trp Asn Gln Met Asn Leu
1               5
```

<210> 4
<211> 9
<212> PRT
<213> Artificial Sequence

<220>
<223> Peptide

<400> 4

```
Cys Tyr Thr Trp Asn Gln Met Asn Leu
1               5
```

<210> 5
<211> 9
<212> PRT
<213> Artificial Sequence

<220>
<223> Peptide

<400> 5

Ala Leu Leu Pro Ala Val Pro Ser Leu
1 5

<210> 6
<211> 9
<212> PRT
<213> Artificial Sequence

<220>
<223> Peptide

<400> 6

Ser Leu Gly Glu Gln Gln Tyr Ser Val
1 5

<210> 7
<211> 9
<212> PRT
<213> Artificial Sequence

<220>
<223> Peptide

<400> 7

Arg Val Pro Gly Val Ala Pro Thr Leu
1 5

<210> 8
<211> 9
<212> PRT
<213> Artificial Sequence

<220>
<223> Peptide

<400> 8

Val Leu Asp Phe Ala Pro Pro Gly Ala
1 5

<210> 9
<211> 10
<212> PRT
<213> Artificial Sequence

<220>
<223> Peptide

<220>
<221> DISULFID
<222> (1)..(2)

<400> 9

Cys Cys Met Thr Trp Asn Gln Met Asn Leu

1 5 10

<210> 10
<211> 10
<212> PRT
<213> Artificial Sequence

<220>
<223> Peptide

<220>
<221> DISULFID
<222> (1)..(2)

<400> 10

Cys Cys Tyr Thr Trp Asn Gln Met Asn Leu
1 5 10

<210> 11
<211> 16
<212> PRT
<213> Artificial Sequence

<220>
<223> Peptide

<400> 11

Lys Arg Tyr Phe Lys Leu Ser His Leu Gln Met His Ser Arg Lys His
1 5 10 15

<210> 12
<211> 19
<212> PRT
<213> Artificial Sequence

<220>
<223> Peptide

<400> 12

Ser Gly Gln Ala Arg Met Phe Pro Asn Ala Pro Tyr Leu Pro Ser Cys
1 5 10 15

Leu Glu Ser

<210> 13
<211> 19
<212> PRT
<213> Artificial Sequence

<220>
<223> Peptide

<400> 13

Arg Ser Asp Glu Leu Val Arg His His Asn Met His Gln Arg Asn Met
1               5                   10                  15

Thr Lys Leu

<210> 14
<211> 22
<212> PRT
<213> Artificial Sequence

<220>
<223> Peptide

<400> 14

Pro Gly Cys Asn Lys Arg Tyr Phe Lys Leu Ser His Leu Gln Met His
1               5                   10                  15

Ser Arg Lys His Thr Gly
            20

<210> 15
<211> 17
<212> PRT
<213> Artificial Sequence

<220>
<223> Peptide

<400> 15

Cys Asn Lys Arg Tyr Phe Lys Leu Ser His Leu Gln Met His Ser Arg
1               5                   10                  15

Lys

<210> 16
<211> 18
<212> PRT
<213> Artificial Sequence

<220>
<223> Peptide

<400> 16

Cys Asn Lys Arg Tyr Phe Lys Leu Ser His Leu Gln Met His Ser Arg
1               5                   10                  15

Lys His

<210> 17
<211> 20
<212> PRT
<213> Artificial Sequence

<220>
<223> Peptide

<400> 17

Cys Asn Lys Arg Tyr Phe Lys Leu Ser His Leu Gln Met His Ser Arg
1 5 10 15

Lys His Thr Gly
20

<210> 18
<211> 18
<212> PRT
<213> Artificial Sequence

<220>
<223> Peptide

<400> 18

Trp Ala Pro Val Leu Asp Phe Ala Pro Pro Gly Ala Ser Ala Tyr Gly
1 5 10 15

Ser Leu

<210> 19
<211> 19
<212> PRT
<213> Artificial Sequence

<220>
<223> Peptide

<400> 19

Cys Trp Ala Pro Val Leu Asp Phe Ala Pro Pro Gly Ala Ser Ala Tyr
1 5 10 15

Gly Ser Leu

<210> 20
<211> 19
<212> PRT
<213> Artificial Sequence

<220>
<223> Peptide

<400> 20

Trp Ala Pro Val Leu Asp Phe Ala Pro Pro Gly Ala Ser Ala Tyr Gly
1               5                   10                  15

Ser Leu Cys

<210> 21
<211> 9
<212> PRT
<213> Artificial Sequence

<220>
<223> Peptide

<400> 21

Arg Tyr Phe Pro Asn Ala Pro Tyr Leu
1               5

<210> 22
<211> 9
<212> PRT
<213> Artificial Sequence

<220>
<223> Peptide

<400> 22

Phe Met Phe Pro Asn Ala Pro Tyr Leu
1               5

<210> 23
<211> 9
<212> PRT
<213> Artificial Sequence

<220>
<223> Peptide

<400> 23

Arg Leu Phe Pro Asn Ala Pro Tyr Leu
1               5

<210> 24
<211> 9
<212> PRT
<213> Artificial Sequence

<220>
<223> Peptide

<400> 24

Arg Met Met Pro Asn Ala Pro Tyr Leu
1               5

<210> 25
<211> 9
<212> PRT
<213> Artificial Sequence

<220>
<223> Peptide

<400> 25

Arg Met Phe Pro Asn Ala Pro Tyr Val
1 5

<210> 26
<211> 9
<212> PRT
<213> Artificial Sequence

<220>
<223> Peptide

<400> 26

Tyr Met Phe Pro Asn Ala Pro Tyr Leu
1 5

<210> 27
<211> 9
<212> PRT
<213> Artificial Sequence

<220>
<223> Peptide

<220>
<221> MISC_FEATURE
<222> (1)..(1)
<223> Xaa is Ser or Ala

<400> 27

Xaa Met Thr Trp Asn Gln Met Asn Leu
1 5

<210> 28
<211> 9
<212> PRT
<213> Artificial Sequence

<220>
<223> Peptide

<220>
<221> MISC_FEATURE
<222> (1)..(1)
<223> Xaa is Ser, Ala, Abu, Arg, Lys, Orn, Cit, Leu, Phe, or Asn

<400> 28

Xaa Tyr Thr Trp Asn Gln Met Asn Leu
1               5

<210> 29
<211> 9
<212> PRT
<213> Artificial Sequence

<220>
<223> Peptide

<400> 29

Ala Tyr Leu Pro Ala Val Pro Ser Leu
1               5

<210> 30
<211> 9
<212> PRT
<213> Artificial Sequence

<220>
<223> Peptide

<400> 30

Phe Leu Gly Glu Gln Gln Tyr Ser Val
1               5

<210> 31
<211> 9
<212> PRT
<213> Artificial Sequence

<220>
<223> Peptide

<400> 31

Ser Met Gly Glu Gln Gln Tyr Ser Val
1               5

<210> 32
<211> 9
<212> PRT
<213> Artificial Sequence

<220>
<223> Peptide

<400> 32

Ser Leu Met Glu Gln Gln Tyr Ser Val
1               5

<210> 33
<211> 9
<212> PRT
<213> Artificial Sequence

<220>
<223> Peptide

<400> 33

Arg Tyr Pro Gly Val Ala Pro Thr Leu
1 5

<210> 34
<211> 16
<212> PRT
<213> Artificial Sequence

<220>
<223> Peptide

<400> 34

Ser Gly Gln Ala Arg Met Phe Pro Asn Ala Pro Tyr Leu Pro Ser Cys
1 5 10 15

<210> 35
<211> 16
<212> PRT
<213> Artificial Sequence

<220>
<223> Peptide

<400> 35

Ser Gly Gln Ala Tyr Met Phe Pro Asn Ala Pro Tyr Leu Pro Ser Cys
1 5 10 15

<210> 36
<211> 19
<212> PRT
<213> Artificial Sequence

<220>
<223> Peptide

<400> 36

Ser Gly Gln Ala Arg Met Phe Pro Asn Ala Pro Tyr Leu Pro Ser Cys
1 5 10 15

Leu Glu Ser

<210> 37
<211> 19
<212> PRT

<213>  Artificial Sequence

<220>
<223>  Peptide

<400>  37

Ser Gly Gln Ala Tyr Met Phe Pro Asn Ala Pro Tyr Leu Pro Ser Cys
1               5                   10                  15

Leu Glu Ser

<210>  38
<211>  19
<212>  PRT
<213>  Artificial Sequence

<220>
<223>  Peptide

<400>  38

Pro Gly Cys Asn Lys Arg Tyr Phe Lys Leu Ser His Leu Gln Met His
1               5                   10                  15

Ser Arg Lys

<210>  39
<211>  20
<212>  PRT
<213>  Artificial Sequence

<220>
<223>  Peptide

<400>  39

Pro Gly Cys Asn Lys Arg Tyr Phe Lys Leu Ser His Leu Gln Met His
1               5                   10                  15

Ser Arg Lys His
            20

<210>  40
<211>  22
<212>  PRT
<213>  Artificial Sequence

<220>
<223>  Peptide

<400>  40

Pro Gly Cys Asn Lys Arg Tyr Phe Lys Leu Ser His Leu Gln Met His
1               5                   10                  15

Ser Arg Lys His Thr Gly
20

## Description

TECHNICAL FIELD

The present invention relates to a lyophilized preparation comprising two or more cancer antigen peptides derived from WT1 protein with an activity of inducing cytotoxic T lymphocytes for cancer peptide vaccine therapy.

BACKGROUND ART

Cancer treatment has evolved with each passing day. Recently, cancer immunotherapy is especially gathering attention as a method of treating cancer. One of the cancer immunotherapies is cancer peptide vaccine therapy. The cancer peptide vaccine therapy is a method of treating cancer which comprises administering a peptide fragment of a highly expressed protein specific to cancer cells to a patient and boosting the patient's immune system to attack the cancer cells. The therapy can select a protein, which is highly expressed in cancer cells and is not expressed in normal cells, and selectively kill only cancer cells, and thus is expected as cancer therapy with high efficacy and safety.

Various killer peptides and helper peptides have been developed as cancer antigen peptides for cancer peptide vaccine therapy, and they have been experimentally used at this stage. However, they have not yet been used as pharmaceutical products (Patent Documents 1, 2, 3 and Non-Patent Document 1).

The present inventors have found a cancer antigen peptide vaccine comprising a killer peptide and a helper peptide for cancer peptide vaccine therapy, and are now studying the vaccine in order to develop pharmaceutical products thereof (Patent Documents 4, 5, 6). Such pharmaceutical products have had some advantages, for example, they have high versatility because a plurality of cancer antigen peptides are contained in single preparation and subjects having human leukocyte antigen(s) (hereinafter, referred to as "HLA(s)") with different generic polymorphisms can be treated with the single preparation, and they can induce helper T cells simultaneously with a plurality of cytotoxic T-cells (hereinafter, referred to as "CTL(s)". The cytotoxic T-cell has the same meanings as the term "cytotoxic T-lymphocyte") because they contain a plurality of killer peptides with helper peptides, and thus can produce a stronger CTL inducing activity.

On the other hand, the dissolution of peptide antigens in a solution for injection was sometimes problematic depending on the amino acid residues of the peptide antigens. Also, in the case of a cocktail vaccine containing a mixture of a plurality of peptide antigens, each peptide antigen composed of various amino acids showed various properties (such as dissolution property by charges). As a result, the development of an optimal preparation capable of dissolving several types of peptides in single solution for injection and efficiently inducing CTL corresponding thereto was often problematic. In addition, some peptides were unstable in solution depending on the amino acid composition of the peptides. Thus, even if the peptides are prepared as a lyophilized preparation, it has been thought that there is a risk of deterioration such as aggregation caused by the dissolution of the peptides. When a lyophilized preparation is prepared from a mixture of several types of the killer peptides and/or the helper peptides described above, there has been some problems, for example, poorly soluble aggregates are formed when water is added into the prepared lyophilized preparation to reconstitute a solution, and analogs derived from helper peptides are generated or increased. Hence, in the current techniques, a method comprising the steps of: preparing lyophilized preparations each containing a killer peptide or a helper peptide in separate vials and then dissolving the lyophilized preparation comprising the helper peptide in water for injection prior to administration, extracting a part of the prepared solution and adding the extracted solution into the lyophilized preparation comprising the killer peptide to prepare a solution, and then adding and mixing an adjuvant thereto at appropriate has been used. The above problems have been overcome by the method. However, the method had some problems, for example, since it was necessary to extemporaneously prepare a solution for administration without preparing the solution in advance in order to prevent the generation of analogs, it was very troublesome to extemporaneously prepare a solution for administration via several steps in medical fields. Also, there has been some problems in terms of manufacturing costs, for example, it is necessary to prepare a plurality of lyophilized preparations in advance, and it is necessary to fill an excess amount of peptides in first dissolution step in view of the loss of peptides in dissolution steps. In addition, it has been assumed that since it is also necessary to lyophilize the two peptides separately to refill the peptides into single vial and to perform the operation under aseptic conditions, the burden on the manufacture field is heavy.

PRIOR ART DOCUMENTS

PATENT DOCUMENTS

Patent Document 1: WO 00/06602
Patent Document 2: WO 00/18795
Patent Document 3: WO 2007/063903
Patent Document 4: WO 2014/157692
Patent Document 5: WO 2010/123065
Patent Document 6: WO 2016/047797

NON-PATENT DOCUMENTS

Non-Patent Document 1: Blood, 2010; 166(2); 177-179.

SUMMARY OF INVENTION

(PROBLEM TO BE SOLVED BY THE INVENTION)

An object of the present invention is to easily prepare a solution for administration comprising two or more peptides for cancer peptide vaccine therapy and formulate the peptides as a mixed lyophilized preparation that can inhibit the generation and increase of analogs of the peptides during long-term storage.

(MEANS FOR SOLVING THE PROBLEMS)

As described in the above BACKGROUND ART, when an aqueous solution comprising two or more peptides for cancer peptide vaccine therapy was lyophilized to prepare single lyophilized preparation, there were some problems, for example, aggregates were formed when the preparation was reconstituted by the addition of water, and analogs of peptides were generated and increased during long-term storage in a mixed lyophilized preparation comprising a plurality of peptides. In addition, since it is long time to reconstitute the mixed lyophilized preparation as an aqueous solution, it has been assumed that the burden on the medical field is heavy. In order to reach the above object, the present inventors have focused on the use of cyclodextrin (CD) and have extensively studied various additives which can be used with the cyclodextrin. As a result, the present inventors have found cancer vaccine preparations which can produce both the inhibition of the generation of aggregates when a lyophilized preparation is reconstituted and the good stability of the preparation during long-term storage with keeping the stability in the preparation process and also shorten the reconstitution time. Based upon the new findings, the present invention has been completed. Particularly, the present inventors have found the lyophilized preparations comprising hydroxypropyl-β-cyclodextrin (HP-β-CD) which generates almost no aggregate when the lyophilized preparation is reconstituted and greatly shortens the reconstitution time. In addition, the present inventors have succeeded that the generation and increase of analogs of the peptides during long-term storage are inhibited by adding suitable amount of methionine in the solution comprising the peptides and lyophilizing the prepared solution.

That is, the summary of the present invention is as follows.

[Item 1] A lyophilized preparation comprising a plurality of peptides and cyclodextrin.

[Item 2] The lyophilized preparation according to the item 1, wherein the plurality of peptides comprises 4, 3 or 2 types pf peptides.

[Item 3] The lyophilized preparation according to the item 1 or 2, wherein the peptide is a cancer antigen peptide.

[Item 4] The lyophilized preparation according to the item 3, wherein the cancer antigen peptide is a killer peptide and/or a helper peptide consisting of a partial peptide consisting of contiguous 7-30 amino acids derived from WT1 protein or a variant thereof.

[Item 5] The lyophilized preparation according to the item 4, wherein the partial peptide comprises a killer peptide and a helper peptide.

[Item 6] The lyophilized preparation according to the item 5, wherein the amounts of the helper peptide and cyclodextrin are 0.1 to 2.0 parts by weight and 0.2 to 5.0 parts by weight, respectively, relative to 1 part by weight of the killer peptide.

[Item 7] The lyophilized preparation according to the item 6, wherein the amounts of the helper peptide and cyclodextrin are 0.5 to 1.0 parts by weight and 0.5 to 2.0 parts by weight, respectively, relative to 1 part by weight of the killer peptide.

[Item 8] The lyophilized preparation according to any one of the items 4-7, wherein the killer peptide is a partial peptide consisting of contiguous 8-10 amino acids in the amino acid sequence of WT1 protein or a variant thereof.

[Item 9] The lyophilized preparation according to the item 8, wherein the killer peptide is a peptide comprising the amino acid sequence selected from the following amino acid sequences:

RMFPNAPYL (SEQ ID NO: 2),
CMTWNQMNL (SEQ ID NO: 3),
CYTWNQMNL (SEQ ID NO: 4),
ALLPAVPSL (SEQ ID NO: 5),
SLGEQQYSV (SEQ ID NO: 6),
RVPGVAPTL (SEQ ID NO: 7),
VLDFAPPGA (SEQ ID NO: 8),
C-CMTWNQMNL (SEQ ID NO: 9) (wherein the bond between C and C is a disulfide bond),
C-CYTWNQMNL (SEQ ID NO: 10) (wherein the bond between C and C is a disulfide bond),

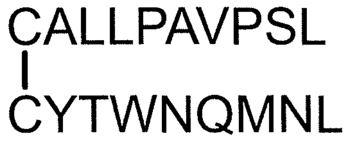 (2) ,

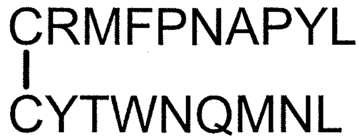 (3) ,

and

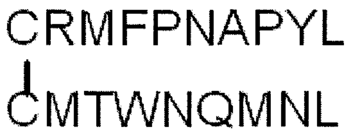 (4) ,

or a peptide comprising an altered amino acid sequence of the amino acid sequence selected from SEQ ID NOs: 2-10 and the formulae (2)-(4) that comprises amino acid alteration in the amino acid sequence and having the CTL inducing activity.

[Item 10] The lyophilized preparation according to the item 9, wherein the killer peptide is a compound of

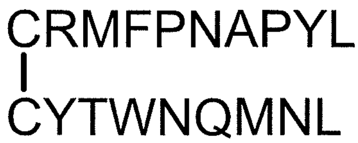 (3)

or

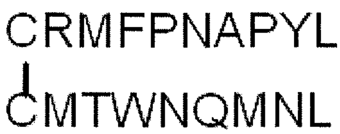 (4) .

[Item 11] The lyophilized preparation according to any one of the items 4-7, wherein the helper peptide is a partial peptide consisting of contiguous 14-30 amino acids in the amino acid sequence of WT1 protein or a variant thereof.

[Item 12] The lyophilized preparation according to the item 11, wherein the helper peptide is a peptide comprising the amino acid sequence selected from the following amino acid sequences:

KRYFKLSHLQMHSRKH (SEQ ID NO: 11),
SGQARMFPNAPYLPSCLES (SEQ ID NO: 12),

RSDELVRHHNMHQRNMTKL (SEQ ID NO: 13),
PGCNKRYFKLSHLQMHSRKHTG (SEQ ID NO: 14),
CNKRYFKLSHLQMHSRK (SEQ ID NO: 15),
CNKRYFKLSHLQMHSRKH (SEQ ID NO: 16),
CNKRYFKLSHLQMHSRKHTG (SEQ ID NO: 17),
WAPVLDFAPPGASAYGSL (SEQ ID NO: 18),
CWAPVLDFAPPGASAYGSL (SEQ ID NO: 19),
WAPVLDFAPPGASAYGSLC (SEQ ID NO: 20),
SGQARMFPNAPYLPSC (SEQ ID NO: 34),
SGQAYMFPNAPYLPSC (SEQ ID NO: 35),
SGQARMFPNAPYLPSCLES (SEQ ID NO: 36),
SGQAYMFPNAPYLPSCLES (SEQ ID NO: 37),
PGCNKRYFKLSHLQMHSRK (SEQ ID NO: 38),
PGCNKRYFKLSHLQMHSRKH (SEQ ID NO: 39), and
PGCNKRYFKLSHLQMHSRKHTG (SEQ ID NO: 40),

or a peptide comprising an altered amino acid sequence of the amino acid sequence selected from SEQ ID NOs: 11-20 and 34-40 that comprises amino acid alteration in the amino acid sequence and having the helper T cell inducing activity.

[Item 13] The lyophilized preparation according to the item 12, wherein the helper peptide is a peptide consisting of the amino acid sequence selected from the following amino acid sequences:

CNKRYFKLSHLQMHSRK (SEQ ID NO: 15),
CNKRYFKLSHLQMHSRKH (SEQ ID NO: 16),
CNKRYFKLSHLQMHSRKHTG (SEQ ID NO: 17),
WAPVLDFAPPGASAYGSL (SEQ ID NO: 18),
CWAPVLDFAPPGASAYGSL (SEQ ID NO: 19) and
WAPVLDFAPPGASAYGSLC (SEQ ID NO: 20).

[Item 14] The lyophilized preparation according to any one of the items 4-7 wherein the killer peptide comprises a compound of formula (3):

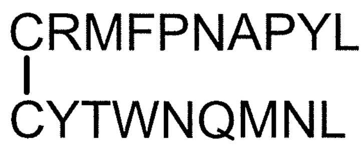
(3)
,

and
the helper peptide comprises a peptide consisting of the amino acid sequence: WAPVLDFAPPGASAYGSL (SEQ ID NO: 18).

[Item 15] The lyophilized preparation according to any one of the items 4-7, wherein the killer peptide comprises a compound of formula (4):

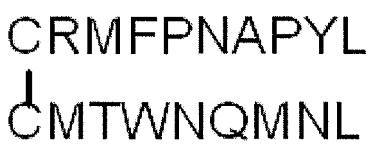
(4)
,

and
the helper peptide comprises a peptide consisting of the amino acid sequence: WAPVLDFAPPGASAYGSL (SEQ ID NO: 18).

[Item 16] The lyophilized preparation according to any one of the items 1-15, wherein the cyclodextrin is one or more selected from the group consisting of α-cyclodextrin (a-CD), β-cyclodextrin (β-CD), γ-cyclodextrin (γ-CD), hydroxyethyl-p-cyclodextrin (HE-β-CD), hydroxypropyl-β-cyclodextrin (HP-β-CD), methyl-β-cyclodextrin (M-β-CD), sulfobutyl ether-β-cyclodextrin (SBE-β-CD), and a pharmaceutically acceptable salt thereof.

[Item 17] The lyophilized preparation according to the item 16, wherein the cyclodextrin is one or more selected from the group consisting of α-cyclodextrin (α-CD), γ-cyclodextrin (γ-CD), hydroxypropyl-β-cyclodextrin(HP-β-CD), and a pharmaceutically acceptable salt thereof.

[Item 18] The lyophilized preparation according to the item 17, wherein the cyclodextrin is hydroxypropyl-β-cyclodextrin (HP-β-CD) or a pharmaceutically acceptable salt thereof.

[Item 19] The lyophilized preparation according to any one of the items 4-7, wherein the killer peptide comprises a compound of formula (3):

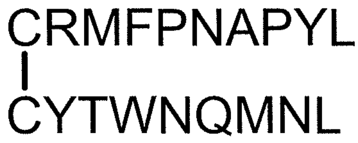
(3)
,

and

the helper peptide comprises a peptide consisting of the amino acid sequence: WAPVLDFAPPGASAYGSL (SEQ ID NO: 18), which further comprises hydroxypropyl-β-cyclodextrin (HP-β-CD) .

[Item 20] The lyophilized preparation according to any one of the items 4-7, wherein the killer peptide comprises a compound of formula (4):

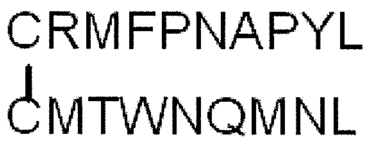
(4)
,

and

the helper peptide comprises a peptide consisting of the amino acid sequence: WAPVLDFAPPGASAYGSL (SEQ ID NO: 18), which further comprises hydroxypropyl-β-cyclodextrin (HP-β-CD) .

[Item 21] The lyophilized preparation according to any one of the items 1-20, which further comprises arginine or methionine.

[Item 22] The lyophilized preparation according to the item 21, the amount of arginine or methionine is 0.01 to 1.0 part by weight relative to 1 part by weight of the helper peptide.

[Item 23] The lyophilized preparation according to the item 22, wherein the amount of arginine or methionine is 0.1 to 0.5 part by weight relative to 1 part by weight of the helper peptide.

[Item 24] The lyophilized preparation according to any one of the items 21-23, which comprises L-methionine as the methionine.

[Item 25] The lyophilized preparation according to any one of the items 1-24, which further comprises an organic acid.

[Item 26] The lyophilized preparation according to the item 25, wherein the amount of the organic acid is 0.01 to 1.0 part by weight relative to 1 part by weight of the helper peptide.

[Item 27] The lyophilized preparation according to the item 26, wherein the amount of the organic acid is 0.05 to 0.5 part by weight relative to 1 part by weight of the helper peptide.

[Item 28] The lyophilized preparation according to any one of the items 25-27, wherein the organic acid is tartaric acid and/or succinic acid.

[Item 29] The lyophilized preparation according to any one of the items 1-28, which further comprises an adjuvant.

[Item 30] The lyophilized preparation according to the item 29, wherein the amount of the adjuvant is 1 to 500 parts by weight relative to 1 part by weight of the killer peptide.

[Item 31] The lyophilized preparation according to the item 29, wherein the amount of the adjuvant is 5 to 100 parts by weight relative to 1 part by weight of the killer peptide.

[Item 32] The lyophilized preparation according to the item 29, wherein the adjuvant is one selected from Freund's adjuvant, Montanide and W/O emulsion.

[Item 33] The lyophilized preparation according to the item 32, wherein the adjuvant is Montanide.

[Item 34] The lyophilized preparation according to any one of the items 4-33 produced by dissolving each ingredient in purified water in an amount of 0.5 to 2 mL per 5 mg of the killer peptide and lyophilizing the solution.

(EFFECTS OF THE INVENTION)

When conventional peptide vaccines comprising a plurality of peptides such as killer peptides and helper peptides are prepared, it was necessary to prepare lyophilized preparations comprising each peptide in separate vials, dissolve each preparation in water and mix the solutions when using the vaccines in terms of the solubility of peptides. On the other hand, in the present invention, cyclodextrin is added into the prepared solution and the solution is lyophilized, and thus one preparation comprising a plurality of peptides can be prepared in a single vial, and a solution for administration can easily be prepared even when the preparation is reconstituted in water.

In addition, the present invention can inhibit the generation and increase of analogs of helper peptides by adding suitable amount of other ingredient(s) such as methionine in the prepared solution and lyophilizing the solution.

DESCRIPTION OF EMBODIMENTS

Hereinafter, the embodiments of the present invention are explained in detail.

The present invention relates to a peptide vaccine preparation comprising two or more antigen peptides.

As used herein, the "cancer antigen peptide" is defined as a peptide which presents on antigen-presenting cells to produce a CTL inducing activity. The "WT1 antigen peptide" means a peptide that has an amino acid sequence derived from WT1 protein, and binds to an MHC class I or MHC class II molecule to be presented on the cell surface in the form of a complex with the MHC molecule and induces killer T cells or helper T cells. As used herein, a WT1 antigen peptide that binds to an MHC class I molecule to induce killer T cells is referred to as "WT1 killer peptide", and a WT1 antigen peptide that binds to an MHC class II molecule to induce helper T cells is referred to as "WT1 helper peptide". The WT1 protein may be mouse or human WT1 protein, but is not limited to thereto. Preferably, the WT1 protein is human WT1 protein. The human WT1 protein has an amino acid sequence of SEQ ID NO: 1.

The WT1 antigen peptide may be modified at a part or all of the amino acid residues in its amino acid sequence. The modified peptides may be prepared by any method known in the art. For example, the modified peptides may be prepared by any modification such as esterification, alkylation, halogenation, phosphorylation, sulfonation and amidation of the functional group(s) in the side chain of the amino acid residues constituting a peptide. Also, a variety of substances may be bound to the peptide at the N- and/or C-terminus. For example, an amino acid, a peptide or an analog thereof may be bound to the peptide. When such substance is bound to the WT1 antigen peptide, the substance may be removed by any process such as an enzymatic reaction *in vivo* or intracellular processing, such that the WT1 antigen peptide is finally generated. The substance may be a substance that regulates the solubility of the peptide, improves the stability of the peptide such as protease resistance, delivers the peptide to a specific tissue or organ, or increases an uptake of the peptide by antigen-presenting cells. The substance may be a substance that increases the CTL inducing activity, for example, a killer or helper peptide other than the WT1 antigen peptide.

The WT1 antigen peptide may comprise a bond other than a peptide bond such as carbon-carbon bond, carbon-nitrogen bond and carbon-sulfur bond. Also, the WT1 antigen peptide may comprise one or more D-amino acids.

The modified peptides described above are illustrative only, and those skilled in the art can easily conceive, prepare, examine and use other variations of the peptides.

As used herein, the "amino acid residue" means a single unit in the amino acids constituting a peptide or a protein. The "amino acid residue" may be a natural or non-natural α-amino acid residue, β-amino acid residue, γ-amino acid residue or δ-amino acid residue. Specifically, the amino acid residue may be a natural α-amino acid residue, ornithine residue, homoserine residue, homocysteine residue, β-alanine, γ-aminobutanoic acid or δ-aminopentanoic acid.

The "amino acid residue" as used herein may be represented as the following abbreviations.

Ala or A: alanine residue
Arg or R: arginine residue
Asn or N: asparagine residue
Asp or D: aspartic acid residue
Cys or C: cysteine residue
Gln or Q: glutamine residue
Glu or E: glutamic acid residue
Gly or G: glycine residue
His or H: histidine residue
Ile or I: isoleucine residue
Leu or L: leucine residue
Lys or K: lysine residue
Met or M: methionine residue
Phe or F: phenylalanine residue

Pro or P: proline residue
Ser or S: serine residue
Thr or T: threonine residue
Trp or W: tryptophan residue
Tyr or Y: tyrosine residue
Val or V: valine residue
Abu: 2-aminobutyric acid residue (also referred to as $\alpha$-aminobutyric acid residue)
Orn: ornithine residue
Cit: citrulline residue

The amino acid sequence of the "peptide" as used herein is described in the conventional manner such that the amino acid residue of the N-terminal amino acid is positioned on the left side, and the amino acid residue of the C-terminal amino acid is positioned on the right side. In the "peptide", unless otherwise indicated, the amino group of the amino acid residue of the N-terminal amino acid binds to hydrogen atom, and the carbonyl group of the amino acid residue of the C-terminal amino acid binds to hydroxyl group. A divalent group of a peptide means a group that binds via the amino group of the amino acid residue of the N-terminal amino acid and the carbonyl group of the amino acid residue of the C-terminal amino acid. In the compound of the present invention, for example, the compounds of formulae (1)-(3), the amino group of the amino acid residue of the N-terminal amino acid in the peptide which is a partial structure thereof binds to hydrogen atom and the carbonyl group of the amino acid residue of the C-terminal amino acid binds to hydroxyl group unless otherwise noted.

MHC in human is referred to as human leukocyte-type antigen (HLA). The HLA corresponding to MHC class I molecule is classified into the subtypes such as HLA-A, B, Cw, F and G. As used herein, the term "MHC class I-restricted" means the property of inducing killer T cells by binding to MHC class I molecule. The "MHC class I-restricted" preferably includes HLA-A-restricted, HLA-B-restricted and HLA-Cw-restricted.

For each subtype of the HLA, polymorphism (allele) has been known. The polymorphism of HLA-A has 27 or more types such as HLA-A1, HLA-A2 and HLA-A24, the polymorphism of HLA-B has 59 or more types such as HLA-B7, HLA-B40 and HLA-B44, and the polymorphism of HLA-Cw has 10 or more types such as HLA-Cw0301, HLA-Cw0401 and HLA-Cw0602. Among the polymorphism, HLA-A2 and HLA-A24 are preferred.

In one embodiment, the WT1 antigen peptide is a WT1 killer peptide, which binds to MHC class I molecule and induces killer T cells (cytotoxic T cells (CTLs)). The WT1 killer peptide induces WT1-specific killer cells when presented on the cell surface in the form of a complex with MHC class I molecule.

In one embodiment, the WT1 killer peptide is a partial peptide consisting of contiguous 7-30 amino acids in the amino acid sequence of human WT1 protein of SEQ ID NO: 1 or a variant thereof. Examples of the WT1 antigen peptide include a peptide comprising the amino acid sequence selected from the following amino sequences:

RMFPNAPYL (SEQ ID NO: 2),
CMTWNQMNL (SEQ ID NO: 3),
CYTWNQMNL (SEQ ID NO: 4),
ALLPAVPSL (SEQ ID NO: 5),
SLGEQQYSV (SEQ ID NO: 6),
RVPGVAPTL (SEQ ID NO: 7), and
VLDFAPPGA (SEQ ID NO: 8),

and a peptide comprising an altered amino acid sequence of the amino acid sequence selected from SEQ ID NOs: 2-8 that comprises amino acid alteration in the amino acid sequence and having the CTL inducing activity, but are not limited thereto. Preferred examples thereof include a peptide consisting of the amino acid sequence selected from SEQ ID NOs: 2-8, and a peptide consisting of an altered amino acid sequence of the amino acid sequence selected from SEQ ID NOs: 2-8 that comprises amino acid alteration in the amino acid sequence and having the CTL inducing activity. More preferred examples thereof include a peptide consisting of the amino acid sequence selected from SEQ ID NOs: 2-8. Furthermore preferred examples thereof include a peptide consisting of the amino acid sequence selected from SEQ ID NOs: 2-6 and 8.

In one embodiment, the WT1 killer peptide is a compound in which cysteine residue is bound to the N-terminal cysteine residue of the partial peptide consisting of contiguous 7-30 amino acids in the amino acid sequence of human WT1 protein of SEQ ID NO: 1 or a variant thereof by a disulfide bond or a pharmaceutically acceptable salt thereof. Examples of the WT1 antigen peptide include a peptide comprising the amino acid sequence selected from the following sequence:

C-CMTWNQMNL (SEQ ID NO: 9) (wherein the bond between C and C is a disulfide bond) or

C-CYTWNQMNL (SEQ ID NO: 10) (wherein the bond between C and C is a disulfide bond),

and a peptide comprising an altered amino acid sequence of the amino acid sequence of SEQ ID NO: 9 or 10 that comprises amino acid alteration in the amino acid sequence and having the CTL inducing activity, but are not limited thereto. Preferred examples thereof include a peptide consisting of the amino acid sequence of SEQ ID NO: 9 or 10 and a peptide consisting of an altered amino acid sequence of the amino acid sequence of SEQ ID NO: 9 or 10 that comprises amino acid alteration in the amino acid sequence and having the CTL inducing activity. More preferred examples thereof include a peptide consisting of the amino acid sequence of SEQ ID NO: 9 or 10. Furthermore preferred examples thereof include a peptide consisting of the amino acid sequence of SEQ ID NO: 10.

As used herein, the "peptide comprising the amino acid sequence" means a peptide in which an additional amino acid(s) is/are added to the N-terminal and/or C-terminal amino acid in the given amino acid sequence as usually understood.

As used herein, the "peptide comprising an altered amino acid sequence of the amino acid sequence ... that comprises amino acid alternation in the amino acid sequence having the CTL inducing activity" is also referred to as "altered killer peptide". The altered killer peptide means a peptide that consists of an amino acid sequence wherein one to several, preferably one to three, more preferably two or one amino acid is deleted from, substituted in, and/or added to the original amino acid sequence, and binds to MHC class I molecule and induces CTLs. The preferred position of the amino acid to be substituted includes the 1-position (N-terminus), the 2-position, the 3-position and the 9-position for a peptide consisting of 9 amino acid residues, but is not limited thereto. The number of amino acids to be added (including "inserted") is preferably 1 or 2, and more preferably 1. The preferred position of the amino acid to be added includes the C-terminus. The number of amino acids to be deleted is preferably 1. In the alteration, the amino acid to be added or substituted may be a non-natural amino acid other than the 20 genetically encoded amino acids.

The regularity of the amino acid sequence (binding motif) has been known in peptides capable of binding to an HLA antigen for each type of polymorphism in an HLA subtype. For example, as to the binding motif for HLA-A24, it has been known that in a peptide consisting of 8 to 11 amino acid residues, the amino acid at the 2-position is Tyr, Phe, Met or Trp, and the amino acid at the C-terminus is Phe, Leu, Ile, Trp or Met (J. Immunol., 152, p3913, 1994; J. Immunol., 155, p4307, 1994; Immunogenetics, 41, p178, 1995). Thus, in a peptide, for example, a peptide consisting of 9 amino acid residues, the amino acid at the 2-position may be replaced with Tyr, Phe, Met or Trp and/or the amino acid at the 9-position may be replaced with Phe, Leu, Ile, Trp or Met. The peptide comprising such amino acid alteration is preferred as an altered killer peptide. Similarly, as to the binding motif for HLA-A2, it has been known that in a peptide consisting of 8 to 11 amino acid residues, the amino acid at the 2-position is Leu or Met, and the amino acid at the C-terminus is Val or Leu. Thus, in a peptide, for example, a peptide consisting of 9 amino acid residues, the amino acid at the 2-position may be replaced with Leu or Met and/or the amino acid at the 9-position may be replaced with Val or Leu. The peptide comprising such amino acid alteration is preferred as an altered killer peptide.

Examples of the altered killer peptide include the following peptides:

an altered killer peptide of RMFPNAPYL (SEQ ID NO: 2) such as

RYFPNAPYL (SEQ ID NO: 21) (WO 03/106682),
FMFPNAPYL (SEQ ID NO: 22),
RLFPNAPYL (SEQ ID NO: 23),
RMMPNAPYL (SEQ ID NO: 24),
RMFPNAPYV (SEQ ID NO: 25), and
YMFPNAPYL (SEQ ID NO: 26) (WO 2009/072610);

an altered killer peptide of CMTWNQMNL (SEQ ID NO: 3) such as
CYTWNQMNL (SEQ ID NO: 4) (WO 02/79253),

Xaa-Met-Thr-Trp-Asn-Gln-Met-Asn-Leu (SEQ ID NO: 27) (wherein Xaa represents Ser or Ala), and
Xaa-Tyr-Thr-Trp-Asn-Gln-Met-Asn-Leu (SEQ ID NO: 28)

(wherein Xaa represents Ser, Ala, Abu, Arg, Lys, Orn, Cit, Leu, Phe or Asn) (WO 2004/026897);
an altered killer peptide of ALLPAVPSL (SEQ ID NO: 5) such as

AYLPAVPSL (SEQ ID NO: 29) (WO 2003/106682);
an altered killer peptide of SLGEQQYSV (SEQ ID NO: 6) such as
FLGEQQYSV (SEQ ID NO: 30),
SMGEQQYSV (SEQ ID NO: 31), and

SLMEQQYSV (SEQ ID NO: 32) (WO 2009/072610); and

an altered killer peptide of RVPGVAPTL (SEQ ID NO: 7) such as RYPGVAPTL (SEQ ID NO: 33) (WO 2003/106682), but are not limited thereto.

In one embodiment, the altered killer peptide is a compound of formula (1):

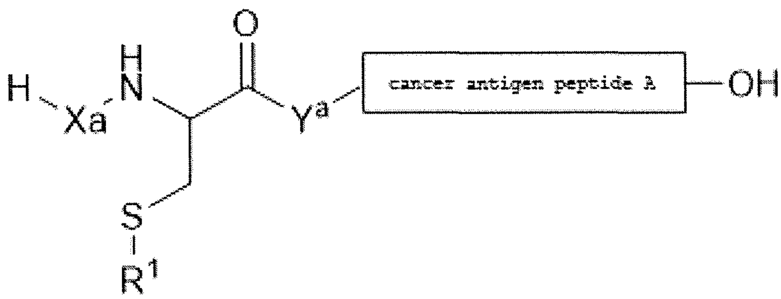

(1)

or a pharmaceutically acceptable salt thereof, wherein Xa and $Y^a$ are single bond,
cancer antigen peptide A is a peptide consisting of the amino acid sequence selected from the group consisting of the following amino acid sequences:

RMFPNAPYL (SEQ ID NO: 2),
ALLPAVPSL (SEQ ID NO: 5),
SLGEQQYSV (SEQ ID NO: 6), and
RVPGVAPTL (SEQ ID NO: 7),

and the amino group of the N-terminal amino acid of the cancer antigen peptide A binds to $Y^a$ in the formula (1), and the carbonyl group of the C-terminal amino acid of the cancer antigen peptide A binds to the hydroxy group in the formula (1) ,
$R^1$ is cancer antigen peptide C,
cancer antigen peptide C is different in sequence from cancer antigen peptide A and is a peptide consisting of the amino acid sequence selected from the group consisting of the following amino acid sequences:

CMTWNQMNL (SEQ ID NO: 3) and
CYTWNQMNL (SEQ ID NO: 4),

and the thioether group of cysteine residue of the cancer antigen peptide C binds to the thioether group in the formula (1), and a compound consisting of an altered compound that comprises amino acid alteration in the compound of formula (1) and having the CTL inducing activity.

Examples of such altered killer peptide include the following compounds:

a compound of formula (2):

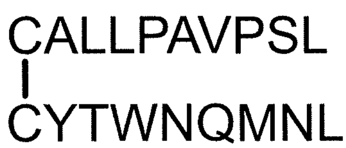

(2)

wherein the bond between C and C is a disulfide bond,
a compound of formula (3):

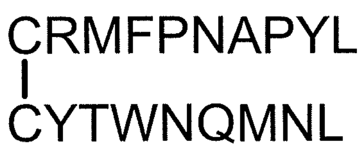

(3)

wherein the bond between C and C is a disulfide bond, and
a compound of formula (4):

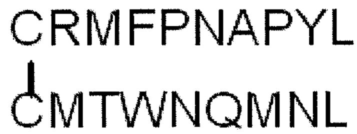

(4)

wherein the bond between C and C is a disulfide bond, and a compound consisting of an altered compound that comprises amino acid alteration in the compound selected from formulae (1) - (4) and having the CTL inducing activity, but are not limited thereto. Preferably, the altered killer peptide is the compound selected from formulae (2) - (4). More preferably, the altered killer peptide is the compound of formula (3) or (4). Furthermore preferably, the altered killer peptide is the compound of formula (3).

In one embodiment, the WT1 antigen peptide is a WT1 helper peptide that binds to MHC class II molecule and induces helper T cells ($CD4^+$ T cells). The WT1 helper peptide induces WT1-specific helper T cells when presented on the cell surface in the form of a complex with MHC class II molecule. The WT1-specific helper T cells produce various cytokines (e.g., IL-2, IL-4, IL-5, IL-6 or interferon (IFN)) and promote the proliferation, differentiation or maturation of B cells and other subsets of T cells. The WT1 helper peptide activates helper T cells to produce the induction or maintenance of the differentiation of CTLs and the activation effect of effector cells such as macrophages, and thus it can be effectively used for the treatment or prophylaxis of cancer.

The HLA corresponding to MHC class II molecule is classified into the subtypes such as HLA-DR, DQ and DP. As used herein, the term "MHC class II-restricted" means the property of inducing helper T cells by binding to MHC class II molecule. The "MHC class II-restricted" preferably includes HLA-DR-restricted, HLA-DQ-restricted and HLA-DP-restricted.

In one embodiment, the WT1 helper peptide is a partial peptide consisting of contiguous 7-30 amino acids, preferably 14-30 amino acids in the amino acid sequence of human WT1 protein of SEQ ID NO: 1 or a variant thereof. Examples of such WT1 helper peptide include a peptide comprising the amino acid sequence selected from the following amino acid sequences:

KRYFKLSHLQMHSRKH (SEQ ID NO: 11),
SGQARMFPNAPYLPSCLES (SEQ ID NO: 12),
RSDELVRHHNMHQRNMTKL (SEQ ID NO: 13),
PGCNKRYFKLSHLQMHSRKHTG (SEQ ID NO: 14),
CNKRYFKLSHLQMHSRK (SEQ ID NO: 15),
CNKRYFKLSHLQMHSRKH (SEQ ID NO: 16),
CNKRYFKLSHLQMHSRKHTG (SEQ ID NO: 17),
WAPVLDFAPPGASAYGSL (SEQ ID NO: 18),
CWAPVLDFAPPGASAYGSL (SEQ ID NO: 19),
WAPVLDFAPPGASAYGSLC (SEQ ID NO: 20),
SGQARMFPNAPYLPSC (SEQ ID NO: 34),
SGQAYMFPNAPYLPSC (SEQ ID NO: 35),
SGQARMFPNAPYLPSCLES (SEQ ID NO: 36),
SGQAYMFPNAPYLPSCLES (SEQ ID NO: 37),
PGCNKRYFKLSHLQMHSRK (SEQ ID NO: 38),
PGCNKRYFKLSHLQMHSRKH (SEQ ID NO: 39), and
PGCNKRYFKLSHLQMHSRKHTG (SEQ ID NO: 40),

and a peptide comprising an altered amino acid sequence of the amino acid sequence selected from SEQ ID NOs: 11-20 and 34-40 that comprises amino acid alteration in the amino acid sequence and having the helper T cell inducing activity, but are not limited thereto. Preferably, the WT 1 helper peptide is a peptide consisting of the amino acid sequence selected from SEQ ID NOs: 11-20 and 34-40, and a peptide comprising an altered amino acid sequence of the amino acid sequence selected from SEQ ID NOs: 11-20 and 34-40 that comprises amino acid alteration in the amino acid and having the helper T cell inducing activity. More preferably, the WT1 helper peptide is a peptide consisting of the amino acid sequence selected from SEQ ID NOs: 11-20 and 34-40. Furthermore preferably, the WT1 helper peptide is a peptide consisting of the amino acid sequence selected from SEQ ID NOs: 11-20.

As used herein, the "peptide comprising an altered amino acid sequence of ... that comprises amino acid

alternation in the amino acid sequence having the helper T cell inducing activity" is also referred to as "altered helper peptide". The altered helper peptide means a peptide that consists of an amino acid sequence wherein one to several, preferably one to three and furthermore preferably two or one amino acid is deleted from, substituted in and/or added to the original amino acid sequence, and binds to MHC class II and induces helper T cells. In the alteration, the amino acid to be added or substituted may be a non-natural amino acid other than the 20 genetically encoded amino acids.

Examples of the altered helper peptide include the following peptides:

an altered helper peptide of SGQARMFPNAPYLPSCLES (SEQ ID NO: 36) such as

SGQAYMFPNAPYLPSCLES (SEQ ID NO: 37) (WO 2007/120673),
SGQARMFPNAPYLPSC (SEQ ID NO: 34) and
SGQAYMFPNAPYLPSC (SEQ ID NO: 35); and

an altered helper peptide of PGCNKRYFKLSHLQMHSRKHTG (SEQ ID NO: 40) such as

PGCNKRYFKLSHLQMHSRK (SEQ ID NO: 38),
PGCNKRYFKLSHLQMHSRKH (SEQ ID NO: 39),
KRYFKLSHLQMHSRKH (SEQ ID NO: 11),
CNKRYFKLSHLQMHSRK (SEQ ID NO: 15),
CNKRYFKLSHLQMHSRKH (SEQ ID NO: 16),
CNKRYFKLSHLQMHSRKHTG (SEQ ID NO: 17),
CWAPVLDFAPPGASAYGSL (SEQ ID NO: 19) and
WAPVLDFAPPGASAYGSLC (SEQ ID NO: 20), but are not limited thereto.

The WT1 antigen peptides (killer peptides and helper peptides) include not only the peptides and compounds described above but also the compounds descried in WO 2000/006602, WO 2002/079253, WO 2003/106682, WO 2004/026897, WO 2004/063903, WO 2007/063903, WO 2010/123065, WO 2014/157692, WO 2018/101309, WO 2005/053618, WO 2007/047764, WO 2007/120673, WO 2005/045027, WO 2010/037395, WO 2000/018795, WO 2002/028414, WO 2003/037060 and WO 2004/100870.

In one embodiment, the preparation of the present invention may be used as a preparation comprising a single cancer antigen peptide or as a preparation comprising a plurality of cancer antigen peptides. Examples of the cancer antigen peptide include a MHC class I cancer antigen peptide derived from a cancer antigen protein selected from the group consisting of MAGE-A1, MEGA-A2, MEGA-A3, MEGA-A4, MEGA-A6, MEGA-A10, MEGA-A12, BAGE, DAM-6, DAM-10, GAGE-1, GAGE-2, GAGE-3, GAGE-4, GAGE-5, GAGE-6, GAGE-7B, GAGE-8, NA88-A, NY-ESO-1, NY-ESO-1a, MART-1/Melan-A, MC1R, Gp100, PSA, PSM, Tyrosinase, Proteinase 3, TRP-1, TRP-2, ART-4, CAMEL, CEA, Ep-CAM, Cyp-B, Her2/neu, VEGFR, hTERT, hTRT, iCE, MUC1, MUC2, PRAME, P15, RU1, RU2, SART-1, SART-2, SART-3, AFP, β-Catenin, Caspase-8, CDK-4, ELF2, GnT-V, G250, HSP70-2M, HST-2, KIAA0205, MUM-1, MUM-2, MUM-3, Myosin, RAGE, TRP-2, 707-AP, Survivin, Livin, SYT-SSX and WT1, and a MHC class II antigen peptide derived from a cancer antigen protein selected from the group consisting of MAGE-A1, MAGE-A2, MAGE-A3, MAGE-A6, NY-ESO-1, MART-1/Melan-A, Gp100, PSA, Tyrosinase, CEA, HER-2/neu, hTERTMUC1, SART-3 and WT1, but are not limited thereto. Preferably, the cancer antigen peptide is a MHC class I cancer antigen peptide derived from WT1 protein or/and a MHC class II cancer antigen peptide derived from WT1 protein.

In one embodiment, the preparation of the present invention may be used as a preparation comprising only a MHC class I antigen peptide derived from a cancer antigen protein or a MHC class II antigen peptide derived from a cancer antigen protein or in combination with the preparation comprising only a MHC class I antigen peptide derived from a cancer antigen protein or a MHC class II antigen peptide derived from a cancer antigen protein.

In one embodiment, the preparation of the present invention may be used as a preparation comprising both a MHC class I antigen peptide derived from a cancer antigen protein and a MHC class II antigen peptide derived from a cancer antigen protein or as a preparation comprising a plurality of MHC class I antigen peptides derived from cancer antigen proteins or a plurality of MHC class II antigen peptides derived from cancer antigen proteins, or in combination with the preparation comprising said MHC class I antigen peptide derived from cancer antigen protein or said MHC class II antigen peptide derived from cancer antigen protein.

In one embodiment, the preparation of the present invention may be used as a preparation comprising only a WT1 killer peptide or a WT1 helper peptide or in combination with the preparation comprising only a WT1 killer peptide or a WT1 helper peptide.

In one embodiment, the preparation of the present invention may be used as a preparation comprising both a WT1 killer peptide and a WT1 helper peptide or as a preparation comprising a plurality of (preferably, four, three, two types of) WT1 killer peptides or WT1 helper peptides, or in combination with the preparation comprising said WT1 killer

peptide or helper peptide.

In one embodiment, the preparation of the present invention may be used as a preparation comprising one type of a WT killer peptide or a WT1 helper peptide or in combination with the preparation comprising the WT1 killer peptide or the WT1 helper peptide.

The amount of the killer peptide used is 0.01 mg to 1000 mg, preferably 0.1 mg to 100 mg, and more preferably 1 mg to 50 mg per vial.

The amount of the helper peptide used is 0.01 mg to 1000 mg, preferably 0.1 mg to 100 mg, and more preferably 1 mg to 50 mg per vial. Alternatively, the amount thereof is 0.1 to 10 parts by weight, preferably 0.3 to 1.5 parts by weight, and more preferably 0.5 to 1.0 part by weight, relative to 1 part by weight of the killer peptide used.

The WT1 antigen peptide may be prepared according to the commonly-used method in peptide synthesis. Examples of the method include methods described in the literatures (e.g., Peptide Synthesis, Interscience, New York, 1966; The Proteins, Vol 2, Academic Press Inc., New York, 1976; Peptide Synthesis, Maruzen Co., Ltd., 1975; Basic and Experiment of Peptide Synthesis, Maruzen Co., Ltd., 1985; and Development of Pharmaceutical Product subsequent Vol.14, Peptide Synthesis, Hirokawa Shoten, 1991). For example, the method includes the Fmoc method or the Boc method using a solid phase synthesizer, or by sequential condensation of Boc-amino acid or Z-amino acid in the liquid phase synthesis (wherein Fmoc is 9-fluorenylmethoxycarbonyl group, Boc is t-butoxycarbonyl group, and Z is benzyloxycarbonyl group).

The functional group in an intermediate for preparing the WT1 antigen peptide such as amino group, carboxy group and mercapto group may be protected by a suitable protecting group or deprotected according to a protection or deprotection technique as appropriate. The suitable protecting group, protection method and deprotection method are specifically described in the literatures such as "Protective Groups in Organic Synthesis 2nd Edition (John Wiley & Sons, Inc.; 1990)". Examples of the protecting group of mercapto group include acetamidomethyl group and trityl group.

When the WT1 antigen peptide has a disulfide bond, the disulfide bond may be formed between two different peptides each containing cysteine residue, or between a peptide containing cysteine residue and cysteine, according to the commonly-used method in peptide chemistry. Examples of the method for forming a disulfide bond include methods described in the literatures (e.g., Peptide Synthesis, Interscience, New York, 1966; The Proteins, Vol 2, Academic Press Inc., New York, 1976; Peptide Synthesis, Maruzen Co., Ltd., 1975; Basic and Experiment of Peptide Synthesis, Maruzen Co., Ltd., 1985; and Development of Pharmaceutical Product subsequent Vol.14, Peptide Synthesis, Hirokawa Shoten, 1991).

Specifically, when the peptide contains one cysteine residue, a compound having a disulfide bond (a disulfide compound) may be prepared by removing all protecting groups including the protecting group of mercapto group on the cysteine side chain and oxidizing the peptide in an inert solvent. Also, the compound may be prepared by mixing two intermediates each having mercapto group in a suitable solvent and oxidizing the mixture. As appropriate, the method for oxidation may be selected from the well-known methods for forming a disulfide bond in general peptide synthesis. Examples thereof include iodine oxidation, air oxidation under alkaline or acidic conditions and oxidation with an oxidant under alkaline or acidic conditions to form a disulfide bond. Examples of the oxidant include iodine, dimethyl sulfoxide (DMSO), and potassium ferricyanide. The solvent used may be water, acetic acid, methanol, chloroform, DMF, DMSO, or a mixture thereof. The oxidation reaction often provides a mixture of symmetric and asymmetric disulfide compounds. The desired asymmetric disulfide compound may be obtained by purification using techniques such as various types of chromatography and recrystallization. Alternatively, a disulfide bond may be selectively formed by mixing an intermediate having activated mercapto group and another intermediate having mercapto group. Examples of the intermediate having activated mercapto group include an intermediate having mercapto group bonded with Npys group (3-nitro-2-pyridinesulphenyl group). Alternatively, one intermediate is mixed with an agent to activate mercapto group, for example, 2,2'-dithiobis(5-nitropyridine), and then the other intermediate is added thereto, whereby a disulfide bond may be selectively formed (Tetrahedron Letters. Vol.37. No.9, pp. 1347-1350) .

The methods described above may be used even when the peptide contains two or more cysteine residues. In this case, isomers having different disulfide bonding patterns are obtained. A desired dimer having a disulfide bond between specific cysteine residues can be obtained by using a specific combination of protecting groups of the cysteine side chains. Examples of the combination of protecting groups include MeBzl (methylbenzyl) group and Acm (acetamidomethyl) group, Trt (trityl) group and Acm group, Npys (3-nitro-2-pyridylthio) group and Acm group, and S-But(S-tert-butyl) group and Acm group. For example, when MeBzl group and Acm group are combined, the MeBzl group and protecting groups other than those of cysteine side chains are removed, a solution comprising a peptide monomer is subjected to air oxidation reaction to form a disulfide bond between the deprotected cysteine residues, and then after the deprotection with iodine and oxidation, a disulfide bond between the cysteine residues protected with the Acm group is formed.

The WT1 antigen peptide may be a peptide composed of a killer peptide and a helper peptide or two different killer peptides or helper peptides by a disulfide bond. Such peptide may be synthesized by the method comprising the following steps (1) to (3).

In the step (1), Fmoc-C(Mmt)A-SBn and a first antigen peptide are used to synthesize a peptide wherein the carbonyl group of the C-terminal amino acid in C(Mmt)A binds to the N-terminal amino group of the first antigen peptide. The term "Fmoc" is 9-fluorenylmethoxycarbonyl group. The term "Mmt" is monomethoxytrityl group. The term "SBn" is thiobenzyl group.

In the step (2), the peptide obtained in said step (1) and a second antigen peptide having one cysteine residue protected by Npys group at the N-terminus are used to synthesize a peptide wherein the thioether group of cysteine residue in the first antigen peptide of the peptide obtained in said step (1) binds to the thioether group of cysteine residue added to the N-terminus of the second antigen peptide. The term "Npys" is 3-nitro-2-pyridylthio group.

In the step (3), the peptide obtained in said step (2) and a third antigen peptide having cysteine residue protected by SPy group to synthesize a peptide wherein the thioether group of N-terminal cysteine residue in the second peptide of the peptide obtained in said step (2) binds to the thioether group of cysteine residue of the third antigen peptide. The term "Spy" is 2-pyridylsulfide group.

The WT1 antigen peptide thus obtained may be purified according to any methods known to a person skilled in the art or the commonly-used method in peptide chemistry. For example, the WT1 antigen peptide may be purified by the techniques such as various types of chromatography (e.g., silica gel column chromatography, ion exchange column chromatography, gel filtration or reversed-phase chromatography) and recrystallization. For example, the recrystallization solvent used may be an alcohol solvent such as methanol, ethanol and 2-propanol, an ether solvent such as diethyl ether, an ester solvent such as ethyl acetate, an aromatic hydrocarbon solvent such as benzene and toluene, a ketone solvent such as acetone, a hydrocarbon solvent such as hexane, an aprotonic solvent such as dimethylformamide and acetonitrile, water, or a mixture thereof. As an alternative purification method, the method described in the literatures such as Jikken Kagaku Kouza (The Chemical Society of Japan ed., Maruzen) Vol.1 may be used.

Purification methods for disulfide compounds are described in the literatures (e.g., Peptide Synthesis, Interscience, New York, 1966; The Proteins, Vol 2, Academic Press Inc., New York, 1976; Peptide Synthesis, Maruzen Co., Ltd., 1975; Basic and Experiment of Peptide Synthesis, Maruzen Co., Ltd., 1985; and Development of Pharmaceutical Product subsequent Vol.14, Peptide Synthesis, Hirokawa Shoten, 1991). Among them, HPLC is preferred.

When the WT1 antigen peptide has one or more asymmetric points, the peptide may be prepared from a starting material (an amino acid) having asymmetric point(s) according to the method commonly used in the art. In addition, in order to improve the optical purity of the WT1 antigen peptide, the methods such as optical resolution method may be performed at a suitable stage of the preparation process. Examples of the optical resolution method include a diastereomer method which forms a salt of the WT1 antigen peptide or an intermediate thereof with an optically active acid (e.g., monocarboxylic acid such as mandelic acid, N-benzyloxyalanine and lactic acid, dicarboxylic acid such as tartaric acid, o-diisopropylidenetartaric acid and malic acid, and sulfonic acid such as camphorsulfonic acid and bromocamphorsulfonic acid) in an inert solvent (e.g., alcohol solvent such as methanol, ethanol and 2-propanol, ether solvent such as diethyl ether, ester solvent such as ethyl acetate, hydrocarbon solvent such as toluene, and aprotonic solvent such as acetnitrile, or a mixture thereof) . When the WT1 antigen peptide or an intermediate thereof has an acidic functional group such as carboxy group, optional resolution method may also be performed by the formation of a salt with an optically active amine (e.g., organic amine such as $\alpha$-phenethylamine, kinin, quinidine, cinchonidine, cinchonine and strychnine).

The temperature for forming the salt is selected from the range of room temperature to the boiling point of the solvent used. In order to improve the optical purity thereof, it is desirable to once raise the temperature to around the boiling point of the solvent used. When a precipitated salt is collected by filtration, the yield may be increased by cooling as appropriate. The optically active acid or amine may be used in an amount of about 0.5-about 2.0 equivalents, preferably about 1 equivalent relative to the substrate used. As appropriate, crystals may be recrystallized in an inert solvent (e.g., alcohol solvent such as methanol, ethanol and 2-propanol, ether solvent such as diethyl ether, ester solvent such as ethyl acetate, hydrocarbon solvent such as toluene, aprotonic solvent such as acetonitrile, a mixture thereof) to provide an optically active salt with high purity. In addition, as appropriate, the optically resolved salt may be treated with an acid or a base according to the conventional method in the art to give a free form thereof.

As used herein, the "pharmaceutically acceptable salt" may be an acid addition salt or a base addition salt. Examples of the acid addition salt include a salt with an inorganic acid salt such as hydrochloride, hydrobromide, sulfate, hydroiodide, nitrate and phosphate, and a salt with an organic acid such as citrate, oxalate, acetate, formate, propionate, benzoate, trifluoroacetate, maleate, tartrate, succinate, methanesulfonate, benzenesulfonate and p-toluenesulfonate, and examples of the base addition salt include a salt with an inorganic base such as sodium salt, potassium salt, calcium salt, magnesium salt and ammonium salt, and a salt with an organic base such as triethylammonium salt, triethanolammonium salt, pyridinium salt and diisopropylammonium salt. In addition, the salt may be a salt with an amino acid (basic or acidic amino acid) such as arginine, aspartic acid and glutamic acid.

The present invention also encompasses a hydrate and a solvate such as ethanol solvate of the WT1 antigen peptide or a pharmaceutically acceptable salt thereof. In addition, the present invention encompasses all possible stereoisomers such as all diastereomers and enantiomers and all types of crystal forms of the WT1 antigen peptide.

The CTL inducing activity of the WT1 antigen peptide can be confirmed by the measurement of the number of

CTLs by the HLA tetramer method (Int. J. Cancer: 100, 565-570 (2002)) or the limiting dilution method (Nat. Med.: 4, 321-327 (1998)). For example, the CTL inducing activity of the HLA-A24-restricted peptide may be confirmed using the HLA-A24 model mouse described in WO 02/47474 and Int. J. Cancer: 100, 565-570 (2002). The helper T cell inducing activity of the WT1 antigen peptide may be confirmed, for example, by any well-known methods such as the method described in Cancer Immunol. Immunother. 51: 271 (2002).

The preparation of the present invention may comprise an ingredient such as, but not limited to, a pharmaceutically acceptable carrier in addition to the active ingredient(s), the WT1 antigen peptide and/or the immunomodulator. In addition, the WT1 antigen peptide in the preparation of the present invention induces WT1-specific CTLs and/or helper T cells, and thus the preparation may comprise or may be administered with a suitable adjuvant to increase the induction efficiency.

In order to effectively acquire cell-mediated immunity, the preparation of the present invention may comprise or may be administered with a suitable adjuvant. The adjuvant such as those described in the literature (Clin. Microbiol. Rev., 7: 277-289, 1994) may be used. Specific examples of the adjuvant include a component derived from fungus, cytokine such as GM-CSF, interleukin-2, interleukin-7 and interleukin-12, a component derived from plant, a component derived from marine organism, mineral gel such as aluminum hydroxide, a surfactant such as lysolecithin and Pluronic polyol, polyanion, peptide and oil emulsion (emulsion preparation). Examples of the component derived from fungus include lipid A, monophosphoryl lipid A which is a derivative of lipid A, dead bacteria of fungus body (e.g. Mycobacterium bacteria such as BCG), a bacterium-derived protein, polynucleotide, Freund's Incomplete Adjuvant, Freund's Complete Adjuvant, cell wall skeleton component (e.g., BCG-CWS) and trehalose dimycolate (TDM).

In addition, the adjuvant may be a sedimentary adjuvant or an oil adjuvant. The sedimentary adjuvant is a suspension of an inorganic substance that absorbs a peptide. Specific examples of the sedimentary adjuvant include sodium hydroxide, aluminum hydroxide (Alum), calcium phosphate, aluminum phosphate, Alum, Pepesu and carboxy vinyl polymer. The oil adjuvant emulsifies an aqueous solution comprising a peptide by forming micelles with mineral oil. Specific examples of the oil adjuvant include liquid paraffin, lanolin, Freund's adjuvant (Freund's complete adjuvant, Freund's incomplete adjuvant), Montanide and W/O emulsion (WO 2006/078059), but are not limited thereto. Preferred examples of Montnide include Montanide ISA51VG.

The amount of the adjuvant in the preparation of the present invention is 1 to 500 parts by weight, preferably 5 to 100 parts by weight, relative to 1 part by weight of the peptide therein.

The preparation of the present invention may comprise or may be administered with a suitable immunomodulator in order to effectively acquire cell-mediated immunity. The immunomodulator such as those described in WO 2016/186177 may be used.

As used herein, the "immunomodulator" means any agent that regulates the transmission of costimulatory signals during the activation of T cells with antigen-presenting cells by interacting with molecules involved in the transmission of the costimulatory signals and present on the antigen-presenting cells and/or T cells, as well as any agent that directly or indirectly regulates the function of molecules involved in the acquirement of immune tolerance (immunosuppression) in the immune system. The "immunomodulator" may be an agent selected from an antibody, a nucleic acid, a protein, a peptide and a low-molecular compound, but is not limited thereto. As used in the context of the "immunomodulator", the term "antibody" includes an antibody fragment. Examples of the antibody fragment include heavy and light chain variable regions of an antibody (VH and VL), F(ab')2, Fab', Fab, Fv, Fd, sdFv and scFV. As used in the context of "immunomodulator", the protein means any protein other than an antibody. Examples of the "immunomodulator" include an immune checkpoint inhibitor, a costimulatory molecule agent, an immune activating agent and a low-molecular inhibitor.

The "immune checkpoint inhibitor" inhibits immunosuppressive effect induced by cancer cells or antigen-presenting cells. Examples of the immune checkpoint inhibitor include an agent against a molecule selected from the group consisting of: (1) CTLA-4 (e.g., ipilimumab and tremelimumab); (2) PD-1 (e.g., nivolumab, pembrolizumab, AMP-224, AMP-514(MEDI0680) and pidilizumab (CT-011)); (3) LAG-3 (e.g., IMP-321 and BMS-986016); (4) BTLA; (5) KIR (e.g., IPH2101); (6) TIM-3; (7) PD-L1 (e.g., durvalumab (MEDI4736), atezolizumab (MPDL3280A), BMS-936559, avelumab (MSB0010718C)); (8) PD-L2; (9) B7-H3 (e.g., MGA-271); (10) B7-H4; (11) HVEM; (12) GAL9; (13) CD160; (14) VISTA; (15) BTNL2; (16) TIGIT; (17) PVR; (18) BTN1A1; (19) BTN2A2; (20) BTN3A2 (Nat Rev Drug Discov. 2013; 12: 130-146; Nikkei Medical Cancer Review 2014; 9; Nat Rev Immunol. 2014; 14: 559-69); and (21) CSF1-R, but are not limited thereto.

The "costimulatory molecule agonist" activates T cells by transmitting an auxiliary signal via a costimulatory molecule on the T cells and/or antigen-presenting cells to attenuate the immunosuppressive effect of cancer cells or antigen-presenting cells. Examples of the costimulatory molecule agonist include an agent against a molecule selected from the group consisting of: (1) 4-1BB; (2) 4-1BB-L; (3) OX40 (4) OX40-L; (5) GITR; (6) CD28; (7) CD40; (8) CD40-L; (9) ICOS; (10) ICOS-L; (11) LIGHT; and (12) CD27, but are not limited thereto.

The "immune activating agent" effectively stimulates killer T cells in the lymph nodes by directly or indirectly activating immune cells such as T cells and dendritic cells. Examples of the immune activating agent include a Toll-like receptor (TLR) agonist, a stimulator of interferon genes (STING) agonist, cytokine, and an agent against heat shock

protein (HSP), but are not limited thereto.

Examples of the "Toll-like receptor (TLR) agonist" include a TLR1/2 agonist, a TLR2 agonist, a TLR3 agonist (e.g., PolyI:C), a TLR4 agonist (e.g., S-type lipopolysaccharide, paclitaxel, lipid A, and monophosphoryl lipid A), a TLR5 agonist (e.g., flagellin), a TLR6/2 agonist (e.g., MALP-2), a TLR7 agonist (e.g., DSP-0509), a TLR7/8 agonist (e.g., gardiquimod, imiquimod, loxoribine, and resiquimod (R848)), a TLR7/9 agonist (e.g., hydroxychloroquine sulfate), a TLR8 agonist (e.g., motolimod (VTX-2337)), a TLR9 agonist (e.g., CpG-ODN) and a TLR11 agonist (e.g., profilin), but are not limited thereto.

Examples of the "cytokine" include IL-1α, IL-1β, IL-2, IL-3, IL-4, IL-5, IL-6, IL-7, IL-8, IL-9, IL-10, IL-11, IL-12, IL-13, IL-14, IL-15, IL-16, IL-17, IL-18, interferon (INF)-α, INF-β, INF-γ, SCF, GM-CSF, G-CSF, M-CSF, erythropoietin, thrombopoietin, MIP (macrophage inflammatory protein) and MCP (monocyte chemoattractant protein), but are not limited thereto.

Examples of the "heat shock protein (HSP)" include HSP70, HSP90, HSP90α, HSP90β, HSP105, HSP72, and HSP40, but are not limited thereto. The agent against HSP includes an HSP inhibitor. Examples of the HSP90 inhibitor include tanespimycin (17-AAG), luminespib (AUY-922, NVP-AUY922), alvespimycin (17-DMAG) hydrochloride, ganetespib (STA-9090), BIIB021 (CNF2024), onalespib (AT13387), geldanamycin, NVP-BEP800, SNX-2112 (PF-04928473), SNX-5422 (PF-4929113), KW-2478, XL888, VER-155008, VER-50589, CH5138303, VER-49009, NMS-E973, PU-H71, HSP990 (NVP-HSP990), and KNK437, but are not limited thereto.

Examples of the "low-molecular inhibitor" include a histone deacetylase inhibitor, a histone demethylase inhibitor, a histone acetyltransferase inhibitor, a histone methyltransferase inhibitor, a DNA methyltransferase inhibitor, an anthracycline antibiotic, a platinum preparation, a MAPK inhibitor, a β-catenin inhibitor, a STAT3 inhibitor, an NF-kB inhibitor, a JAK inhibitor, an mTOR inhibitor, an IDO inhibitor, a COX-2 inhibitor, a CXCR4 inhibitor and an arginase inhibitor, but are not limited thereto.

Examples of the "histone deacetylase inhibitor" include vorinostat (SAHA, MK0683), entinostat (MS-275), panobinostat (LBH589), trichostatin A (TSA), mocetinostat (MGCD0103), BG45, BRD73954, belinostat (PXD101), romidepsin (FK228, depsipeptide), Domatinostat (4SC-202), HPOB, LMK-235, CAY10603, tasquinimod, TMP269, Nexturastat A, Rocilinostat (ACY-1215), RGFP966, RG2833 (RGFP109), Scriptaid, tubastatin A, Pracinostat (SB939), CUDC-101, M344, PCI-34051, dacinostat (LAQ824), tubastatin A hydrochloride, abexinostat (PCI-24781), CUDC-907, AR-42, sodium phenylbutyrate, resminostat, tubacin, xinostat (JNJ-26481585) dihydrochloride, MC1568, givinostat (ITF2357), droxinostat, chidamide (C S055, HBI-8000), CHR-2485, CHR-3996, DAC-060, FRM-0334 (EVP-0334), MGCD-290, CXD-101 (AZD-9468), CG200745, arginine butyrate, sulforaphane, SHP-141, CUDC-907, YM753 (OBP-801), sodium valproate, apicidin (OSI2040) and CI994 (Tacedinaline), but are not limited thereto.

Examples of the "histone demethylase inhibitor" include GSKJ4 HCl, OG-L002, JIB-04, IOX1, SP2509, ORY-1001 (RG-6016), GSK J1, ML324 and GSK-LSD1 2HCl, but are not limited thereto.

Examples of the "histone acetyltransferase inhibitor" include C646, MG149, Remodelin and Anacardic Acid, but are not limited thereto.

Examples of the "histone methyltransferase inhibitor" include Pinometostat (EPZ5676), EPZ005678, GSK343, BIX01294, Tazemetostat (EPZ6438), 3-deazaneplanocin A (DZNeP) HCl, UNC1999, MM-102, SGC0946, entacapone, EPZ015666, UNC0379, EI1, MI-2 (Menin-MLL Inhibitor), MI-3 (Menin-MLL Inhibitor), PFI-2, GSK126, EPZ04777, BRD4770, GSK-2816126, and UNC0631, but are not limited thereto.

Examples of the "DNA methyltransferase inhibitor include decitabine, azacitidine, RG108, thioguanine, zebularine, SGI-110, CC-486, SGI-1027, lomeguatrib and procainamide hydrochloride, but are not limited thereto.

The "anthracycline antibiotic" is intercalated between DNA strands to inhibit DNA relaxation. Examples of the anthracycline antibiotic include doxorubicin, liposomal doxorubicin, daunorubicin, pirarubicin, epirubicin, idarubicin, aclarubicin, amrubicin, aloin and mitoxantrone, but are not limited thereto.

Examples of the "platinum preparation" include cisplatin, carboplatin, miboplatin, nedaplatin, satraplatin (JM-126), oxaliplatin (ELOXATIN), triplatin tetranitrate and a DDS preparation thereof, but are not limited thereto.

Examples of the "MAPK inhibitor" include SB203580, doramapimod (BIRB796), SB202190 (FHPI), LY2228820, VX-702, SB239063, Pexmetinib (ARRY-614), PH-797804, VX-745 and TAK-715, but are not limited thereto.

Examples of the "β-catenin inhibitor" include XAV-939, ICG-001, IWR-1-endo, Wnt-C59 (C59), LGK-974, KY02111, IWP-2, IWP-L6, WIKI4 and FH535, but are not limited thereto.

Examples of the "STAT3 inhibitor" include S3I-201, Stattic, niclosamide, nifuroxazide, napabucasin (BBI608), cryptotanshinone, HO-3867, WHI-P154, FLLL32, STA-21, WP1066, DSP-0337 and SH-4-54, but are not limited thereto.

Examples of the "NF-kB inhibitor" include QNZ (EVP4593), sodium 4-aminosalicylate, JSH-23, phenethyl caffeate, sodium salicylate, andrographolide and SC75741, but are not limited thereto.

Examples of the "JAK inhibitor" include ruxolitinib (INCB018424), tofacitinib (CP-690550) citrate, AZD1480, fedratinib (SAR302503, TG101348), AT9283, tyrphostin B42 (AG-490), momelotinib (CYT387), tofacitinib (CP-690550, tasocitinib), WP1066, TG101209, gandotinib (LY2784544), NVP-BSK805 2HCl, baricitinib (LY3009104, INCB02850), AZ960, CEP-33779, pacritinib (SB1518), WHI-P154, XL019, S-ruxolitinib (INCB018424), ZM39923 HCl, decernotinib

(VX-509), Cerdulatinib (PRT062070, PRT2070), filgotinib (GLPG0634), FLLL32, peficitinib (ASP015K, JNJ-54781532), GLPG0634 analogue, Go6976 and Curcumol, but are not limited thereto.

Examples of the "mTOR inhibitor" include sirolimus (rapamycin), deforolimus (AP23573, MK-8669), everolimus (RAD-001), temsirolimus (CCI-779, NSC683864), zotarolimus (ABT-578), biolimus A9 (umirolimus), AZD8055, KU-0063794, voxtalisib (XL765, SAR245409), MHY1485, dactolisib (BEZ235, NVP-BEZ235), PI-103 and Torkinib (PP242), but are not limited thereto.

Examples of the "IDO inhibitor" include NLG919, INCB024360 analog, indoximod (NLG-8189) and Epacadostat (INCB024360), but are not limited thereto.

Examples of the "COX-2 inhibitor" include valdecoxib, rofecoxib, carprofen, celecoxib, lumiracoxib, tolfenamic acid, nimesulide, niflumic acid, Asaraldehyde, lornoxicam, sodium meclofenamate, amfenac sodium hydrate, diclofenac sodium, ketoprofen, ketorolac, naproxen sodium, indomethacin, ibuprofen, aspirin, mefenamic acid, bromfenac sodium, oxaprozin, zaltoprofen and nepafenac, but are not limited thereto.

Examples of the "CXCR4 inhibitor" include WZ811, Plerixafor (AMD3100) and Plerixafor 8HCl (AMD3100 8HCl), but are not limited thereto.

The WT1 antigen peptide of the present invention produces excellent anti-cancer effects even when combined with an immunomodulator. The effects can be further enhanced or the QOL of patients can be improved by combining the WT1 antigen peptide with at least one additional drug (multidrug therapy).

The WT1 antigen peptide used herein may be used in combination with one or more agents selected from the group consisting of "hormone therapy agent", "immunotherapeutic agent", "biological preparation", "cell growth factor", "cell growth factor inhibitor", "cell growth factor receptor inhibitor", "radiotherapeutic agent", "auxiliary agent" and "chemotherapeutic agent". Preferably, the WT1 antigen peptide may be used in combination with one to five agents selected from said group. More preferably, the WT1 antigen peptide may be used in combination with one to three agents selected from said group. Particularly preferably, the WT1 antigen peptide may be used in combination with one agent selected from said group. Hereinafter, the agent that may be combined with the WT1 antigen peptide and the immunomodulator is referred to as "concomitant drug". The dose of the concomitant drug may be appropriately determined on the basis of the dose used in clinical practice.

The "hormone therapy agent" includes an adrenal cortical hormone agent (e.g., steroidal anti-inflammatory agent, estrogen preparation, progesterone preparation and androgen preparation), an anti-estrogen agent, an estrogen-controlling agent, an estrogen synthesis inhibitor, an anti-androgen agent, an androgen-controlling agent, an androgen synthesis inhibitor, an LH-RH agonist preparation, an LH-RH antagonist preparation, an aromatase inhibitor, a steroid-lactonase inhibitor, a pill preparation, a retinoid and an agent which delays the retinoid metabolism.

Examples of the "hormone therapy agent" include fosfestrol, diethylstilbestrol, fluoxymesterol, chlorotrianisene, methyl testosterone, medroxyprogesterone acetate, megestrol acetate, chlormadinone acetate, cyproterone acetate, danazol, allylestrenol, gestrinone, mepartricin, raloxifene, ormeloxifene, levormeloxifene, tamoxifen citrate, toremifene citrate, iodoxyfene, a pill preparation, mepitiostane, testololactone, aminoglutethimide, goserelin acetate, buserelin, leuprorelin, leuprolide, droloxifene, epitiostanol, ethinylestradiol sulfonate, estramustine, fadrozole hydrochloride, anastrozole, terorazole, ketoconazole, letrozole, exemestane, vorozole, formestane, flutamide, bicalutamide, nilutamide, enzalutamide, mifepristone, finasteride, dexamethasone, prednisolone, betamethasone, triamcinolone, abiraterone, liarozole, bexarotene and DN101, but are not limited thereto.

Examples of the "immunotherapeutic agent" include picibanil, krestin, sizofiran, lentinan, ubenimex, interferon (IFN)-$\alpha$, interferon (IFN)-$\beta$, interferon (IFN)-$\gamma$, interleukin, macrophage colony-stimulating factor, granulocyte colony-stimulating factor, erythropoietin, lymphotoxin, BCG vaccine, corynebacterium parvum, levamisole, polysaccharide K, procodazole, anti-CTLA4 antibody, anti-PD-1 antibody and a TLR agonist (e.g., TLR7 agonist, TLR8 agonist, TLR9 agonist).

Examples of the "biological preparation" include interleukin-2 (Aldesleukin), interferon-$\alpha$, interferon-$\beta$, interferon-$\gamma$, erythropoietin (EPO), granulocyte colony-stimulating factor (filgrastim), granulocyte macrophage colony-stimulating factor (sargramostim), IL13-PE38QQR, Bacille Calmette-Guerin, levamisole, octreotide, CPG7909, Provenge, GVAX, Myvax, Favld, lenalidomide, trastuzumab, rituximab, gemtuzumab ozogamicin, alemtuzumab, endostatin, ibritumomab tiuxetan, tositumomab, cetuximab, zanolimumab, ofatumumab, HGS-ETR1, pertuzumab, M200, SGN-30, matuzumab, adecatumumab, denosumab, zalutumumab, MDX-060, nimotuzumab, MORAb-003, Vitaxin, MDX-101, MDX-010, DPC4 antibody, NF-1 antibody, NF-2 antibody, Rb antibody, p53 antibody, WT1 antibody, BRCA1 antibody, BRCA2 antibody, ganglioside (GM2), prostate specific antigen (PSA), $\alpha$-fetoprotein (AFP), carcinoembryonic antigen (CEA), melanoma-associated antigen (MART-1, gap100, MAGE 1, 3 tyrosine), papilloma virus E6 and E7 fragments and a DDS preparation thereof, but are not limited thereto.

As for the "cell growth factor", "cell growth factor inhibitor" and "cell growth factor receptor inhibitor", the cell growth factor is not particularly limited as long as it is a substance that promotes the cell proliferation. For example, the cell growth factor may be a peptide that has a molecular weight of 20,000 or less and can produce the effect at low concentration through binding to a receptor.

Examples of the "cell growth factor" include Epidermal Growth Factor (EGF), Insulin-Like Growth Factor (IGF (e.g., insulin, IGF-1, IGF-2)), Transforming Growth Factor (TGF (e.g., TGF-$\alpha$, TGF-$\beta$)), Nerve Growth Factor (NGF), Brain-derived Neurotrophic Factor (BDNF), Vascular Endothelial Growth Factor (VEGF), Colony Stimulating Factor (CSF (e.g., Granulocyte-Colony Stimulating Factor (G-CSF)), Granulocyte-Macrophage-Colony Stimulating Factor (GM-CSF)), Platelet-Derived Growth Factor (PDGF), Erythropoietin (EPO), Fibroblast Growth Factor (FGF (e.g., acidic FGF, basic FGF, Keratinocyte Growth Factor (KGK), FGF-10)), Hepatocyte Growth Factor (HGF), heregulin and angiopoietin, but are not limited thereto. The cell growth factor has the same meanings as the term "growth factor".

Examples of the "cell growth factor inhibitor" include epidermal growth factor inhibitor (EGF inhibitor), insulin-like growth factor inhibitor (IGF inhibitor), nerve growth factor inhibitor (NGF inhibitor), brain-derived neurotrophic factor inhibitor (BDNF inhibitor), vesicular endothelial growth factor inhibitor (VEGF inhibitor), colony stimulating factor inhibitor (CSF inhibitor), platelet-derived growth factor inhibitor (PDGF inhibitor), erythropoietin inhibitor (EPO inhibitor), fibroblast growth factor inhibitor (FGF inhibitor), hepatocyte growth factor inhibitor (HGF inhibitor), heregulin inhibitor and angiopoietin inhibitor, but are not limited thereto. The cell growth factor inhibitor has the same meanings as the term "growth factor inhibitor".

Examples of the "cell growth factor receptor inhibitor" include epidermal growth factor receptor inhibitor (EGFR inhibitor), insulin-like growth factor receptor inhibitor (IGFR inhibitor), nerve growth factor receptor inhibitor (NGFR inhibitor), brain-derived neurotrophic factor receptor inhibitor (BDNFR inhibitor), vascular endothelial cell growth factor inhibitor (VEGF inhibitor), colony stimulating factor inhibitor (CSF inhibitor), platelet-derived growth factor receptor inhibitor (PDGFR inhibitor), erythropoietin receptor inhibitor (EPOR inhibitor), fibroblast growth factor receptor inhibitor (FGFR inhibitor), hepatocyte growth factor receptor inhibitor (HGFR inhibitor), heregulin receptor inhibitor and angiopoietin receptor inhibitor, but are not limited thereto. The cell growth factor receptor inhibitor has the same meanings as the term "growth factor receptor inhibitor".

Examples of the "radiotherapeutic agent" include a radioactive material and a radiosensitizer, but are not limited thereto.

The "auxiliary agent" is used for suppressing a side effect caused by an anticancer agent or vomiting. Examples of the auxiliary agent include aprepitant, ondansetron, lorazepam, dexamethasone, diphenhydramine, ranitidine, cimetidine, ranitidine, famotidine, cimetidine, procrit, epoetin alfa, filgrastim, oprelvekin, leucovorin and granulocyte-macrophage-colony stimulating factor (GM-CSF), but are not limited thereto.

Examples of the "chemotherapeutic agent" include an alkylating agent, a platinum preparation, an antimetabolite, a topoisomerase inhibitor, a DNA intercalator, an antimitotic agent, an antitumor antibiotic, a plant-derived anticancer agent, an epigenome drug, an immunomodulator, a molecular targeted therapeutic agent, an angiogenesis inhibitor and other chemotherapeutic agent, but are not limited thereto. Typical examples thereof are listed below.

Examples of the "alkylating agent" include nitrogen mustard, nitrogen mustard N-oxide hydrochloride, chlorambucil, cyclophosphamide, ifosfamide, thiotepa, carboquone, improsulfan tosylate, busulfan, nimustine hydrochloride, mitobronitol, melphalan, dacarbazine, procarbazine, ranimustine, estramustine sodium phosphate, triethylenemelamine, carmustine, lomustine, streptozocin, pipobroman, etoglucid, altretamine, ambamustine, dibrospidium hydrochloride, fotemustine, prednimustine, bendamustine, uramustine, semustine, pumitepa, ribomustin, temozolomide, treosulfan, trofosfamide, zinostatin stimalamer, adozelesin, cystemustine, bizelesin, mechlorethamine, uracil mustard, trabectedin, chlormethine, mannosulfan, triaziquone, procarbazine, canfosfamide, nitrosoureas and a DDS preparation thereof, but are not limited thereto.

Examples of the "platinum preparation" include cisplatin, carboplatin, miboplatin, nedaplatin, satraplatin, oxaliplatin, triplatin tetranitrate and a DDS preparation thereof, but are not limited thereto.

Examples of the "antimetabolite" include an antifolate, a pyrimidine metabolism inhibitor, a purine metabolism inhibitor, a ribonucleotide reductase inhibitor and nucleotide analog, but are not limited thereto.

Examples of the "antimetabolite" include mercaptopurine, 6-mercaptopurine riboside, thioinosine, methotrexate, pemetrexed, eoshitabin, enocitabine, cytarabine, cytarabine ocfosfate, ancitabine hydrochloride, a 5-FU agent (e.g., fluorouracil, carzonal, bennan, lunachol, lunapon, tegafur, tegafur-uracil, tegafur-gimeracil-oteracil potassium (TS-1), UFT, doxifluridine, carmofur, gallocitabine, emitefur and capecitabine), aminopterin, nelarabine, leucovorin calcium, tabloid, butocine, folinate calcium, levofolinate calcium, cladribine, emitefur, fludarabine, gemcitabine, hydroxycarbamide, pentostatin, piritrexim, idoxuridine, mitoguazone, tiazofurine, ambamustine, bendamustine, floxuridine, leucovorin, hydroxyurea, thioguanine, asparaginase, bortezomib, raltitrexed, clofarabine, enocitabine, sapacitabine, azacytidine, sulfadiazine, sulfamethoxazole, trimethoprim, Liproxstatin-1, D4476, Xanthohumol, Epacadostat (INCB024360), Vidofludimus, P7C3, GMX1778 (CHS828), NCT-501, SWO33291, Ro61-8048 and a DDS preparation thereof, but are not limited thereto.

Examples of the "topoisomerase inhibitor" include doxorubicin, daunorubicin, epirubicin, idarubicin, anthracenedione, mitoxantrone, mitomycin C, bleomycin, dactinomycin, plicatomycin, irinotecan, camptothecin, rubitecan, belotecan, etoposide, teniposide, topotecan, amsacrine and a DDS preparation thereof, but are not limited thereto.

Examples of the "DNA intercalator" include proflavine, doxorubicin (adriamycin), daunorubicin, dactinomycin,

thalidomide and a DDS preparation thereof, but are not limited thereto.

Examples of the "antimitotic agent" include paclitaxel, a paclitaxel derivative (e.g., DHA paclitaxel, paclitaxel polyglutamate, nab-paclitaxel, micellar paclitaxel, 7$\alpha$-glucosyloxyacetylpaclitaxel and BMS-275183), docetaxel, vinorelbine, vincristine, vinblastine, vindesine, vinzolidine, etoposide, teniposide, ixabepilone, larotaxel, ortataxel, tesetaxel, ispinesib, colchicine, vinflunine and a DDS preparation thereof, but are not limited thereto.

Examples of the "antitumor antibiotic" include actinomycin D, actinomycin C, mitomycin C, chromomycin A3, mithramycin A, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, amrubicin hydrochloride, neocarzinostatin, zinostatin stimalamer, mithramycin, sarkomycin, carzinophilin, mitotane, zorubicin hydrochloride, mitoxantrone hydrochloride, idarubicin hydrochloride, liposomal doxorubicin and a DDS preparation thereof, but are not limited thereto.

Examples of the "plant-derived anticancer agent" include irinotecan, nogitecan, etoposide, etoposide phosphate, eribulin, sobuzoxane, vinblastine sulfate, vincristine sulfate, vindesine sulfate, teniposide, paclitaxel, paclitaxel injection, docetaxel, DJ-927, vinorelbine, topotecan and a DDS preparation thereof, but are not limited thereto.

Examples of the "epigenome drug" include a DNA methylation inhibitor, a histone deacetylase (HDAC) inhibitor, a DNA methyl transferase (DNMT) inhibitor, a histone deacetylase activator, a histone demethylase inhibitor and methylated nucleotide, but are not limited thereto.

Examples of the "epigenome drug" include vorinostat, belinostat, mocetinostat (MGCD0103), entinostat (SNDX-275), romidepsin, azacytidine, decitabine, GSK2879552 2HI, SGC707, ORY-1001 (RG-6016), PFI-4, SirReal2, GSK2801, CPI-360, GSK503, AMI-1, CPI-169 and a DDS preparation thereof, but are not limited thereto.

Examples of the "immunomodulator" include thalidomide, lenalidomide, pomalidomide and a DDS preparation thereof, but are not limited thereto.

The "molecular targeted therapeutic agent" may be a low-molecular compound or an antibody. Examples of the "molecular targeted therapeutic agent" include a kinase inhibitor, a proteasome inhibitor, a monoclonal antibody, an mTOR inhibitor, a TNF inhibitor and a T-cell inhibitor, but are not limited thereto.

Examples of the "kinase inhibitor" include a tyrosine kinase inhibitor, a serine/threonine kinase inhibitor, a Raf kinase inhibitor, a CDK (cyclin-dependent kinase) inhibitor and a MEK (mitogen-activated protein kinase) inhibitor, but are not limited thereto.

Specific examples of the "kinase inhibitor" include imatinib, gefitinib, erlotinib, afatinib, dasatinib, bosutinib, vandetanib, sunitinib, axitinib, pazopanib, lenvatinib, lapatinib, nintedanib, nilotinib, crizotinib, ceritinib, alectinib, ruxolitinib, tofacitinib, ibrutinib, sorafenib, vemurafenib, dabrafenib, palbociclib, trametinib, regorafenib, cedivanib, lestaurtinib, bandetinib, vatalanib, seliciclib, tivantinib, canertinib, pelitinib, tesevatinib, cediranib, motesanib, midostaurin, foretinib, cabozantinib, selumetinib, neratinib, volasertib, saracatinib, enzastaurin, tandutinib, semaxanib, alvocidib, ICR-62, AEE788, PD0325901, PD153035, TK787, amcasertib (BBI503), E6201, E7050, alvocidib (DSP-2033), TP-0903, TP-0184 and a DDS preparation thereof.

Examples of the "proteasome inhibitor" include bortezomib, carfilzomib and a DDS preparation thereof, but are not limited thereto.

Examples of the "monoclonal antibody" include anti-CD22 antibody, anti-CD20 antibody, anti-CD25 antibody, anti-CD30 antibody, anti-CD33 antibody, anti-CD5 antibody, anti-CD52 antibody, anti-epidermal growth factor receptor antibody (EGFR antibody), anti-vascular endothelial cell growth factor antibody (VEGF antibody), anti-TNF-a antibody, anti-IL-1 receptor antibody, anti-IL-2 receptor antibody, anti-IL-5 receptor antibody, anti-IL-6 receptor antibody, anti-HER2 antibody, anti-IgE antibody, anti-IgG antibody, anti-RS virus antibody, anti-CCR4 antibody, anti-cytotoxic T lymphocyte-associated antigen 4 (CTLA-4, CD152) antibody, anti-PD-1 antibody, anti-receptor activator of nuclear factor $\kappa$B ligand (RANKL) antibody, anti-c-Met antibody and anti-CXCR4 antibody, but are not limited thereto.

Specific examples of the "monoclonal antibody" include ibritumomab tiuxetan, rituximab, cetuximab, infliximab, basiliximab, brentuximab vedotin, tocilizumab, trastuzumab, bevacizumab, omalizumab, mepolizumab, gemtuzumab, ozogamicin, palivizumab, ranibizumab, certolizumab, ocrelizumab, mogamulizumab, eculizumab, pertuzumab, alemtuzumab, inotuzumab, panitumumab, ofatumumab, golimumab, adalimumab, ramucirumab, nivolumab, anakinra, denosumab, ipilimumab, pembrolizumab, matuzumab, farletuzumab, MORAb-004, MORA-b009 and a DDS preparation thereof, but are not limited thereto.

Examples of the "mTOR inhibitor" include everolimus (RAD001), rapamycin (sirolimus), AZD8055, temsirolimus (CCI-779, NSC683864), KU-0063794, voxtalisib (XL-765, SAR245409), MHY1485, dactolisib (BEZ235), PI-103, Torkinib (PP242), ridaforolimus (deforolimus, MK-8669), INK-128 (MLN0128), Torin1, omipalisib (GSK2126458, GSK458), OSI-027, PF-04691502, apitolisib (GDC-0980, RG7422), GSK1059615, gedatolisib (PF-05212384, PKI-587), WYE-132, PP121, WYE-354, AZD2014, Torin2, WYE-687, CH5132799, WAY-600, ETP-46464, GDC-0349, XL388, zotarolimus (ABT-578), tacrolimus (FK506), BGT226 (NVP-BGT226), Palomid 529 (P529), chrysophanic acid and a DDS preparation thereof, but are not limited thereto.

Examples of the "TNF inhibitor" include etanercept, lenalidomide (CC-5013), pomalidomide, thalidomide,

necrostatin-1 and QNZ (EVP4593), but are not limited thereto.

Examples of the "T-cell inhibitor" include abatacept, but are not limited thereto.

Examples of the "angiogenesis inhibitor" include CM101, IFN-α, IL-12, platelet factor-4, suramin, semaxanib, thrombospondin, a VEGFR antagonist, a combination of an angiostatic steroid and heparin, a cartilage-derived angiogenesis inhibitor, a matrix metalloproteinase inhibitor, batimastat, marimastat, angiostatin, endostatin, 2-methoxyestradiol, tecogalan, thrombospondin, an αVβ3 inhibitor, linomide, ADH-1, E7820 and a DDS preparation thereof, but are not limited thereto.

Examples of the "other chemotherapeutic agent" include finasteride, sobuzoxane, obatoclax, efaproxiral, tipifarnib and lonafarnib, but are not limited thereto.

The preparation of the present invention may comprise, but not limited to, a pH adjusting agent, a diluent, a buffering agent, a suspending agent, an emulsifier, a wetting agent, a solubilizer, a dispersant, a preservative and/or a coloring agent used in conventional pharmaceutical preparations, selected from the group consisting of a sugar alcohol such as mannitol, trehalose and lactose; an acid such as hydrochloric acid, sulfuric acid, nitric acid, acetic acid, citric acid, tartaric acid, succinic acid, lactic acid, maleic acid and phosphoric acid; an alkali such as sodium hydroxide, potassium hydroxide and ammonia water; a salt such as sodium chloride, sodium acetate, sodium acetate hydrate, anhydrous sodium acetate, sodium citrate hydrate, sodium dihydrogen citrate, sodium tartrate, sodium succinate, disodium phosphate, dipotassium phosphate, sodium dihydrogen phosphate, potassium dihydrogen phosphate and trisodium phosphate; a synthetic surfactant such as Polysorbate 20, Polysorbate 60 and Polysorbate 80; and a polyethylene glycol such as macrogol and polyethylene glycol (PEG) (e.g., PEG 4000) . Preferred examples thereof include mannitol, trehalose, tartaric acid and succinic acid. More preferred examples thereof include D-mannitol, trehalose hydrate, tartaric acid and succinic acid.

In the preparation of the present invention, the amount of the pH adjusting agent, the diluent, the buffering agent, the suspending agent, the emulsifier, the wetting agent, the solubilizer, the dispersant, the preservative or the coloring agent is 0.001 to 1000 parts by weight, preferably 0.01 to 10 parts by weight, and more preferably 0.1 to 5 parts by weight, relative to 1 part by weight of the peptide used.

The preparation of the present invention may comprise, for example, one or more amino acids selected from an amino acid such as threonine, tryptophan, lysine, hydroxylysine, histidine, arginine, cysteine, cystine and methionine, a derivative of said amino acid or a pharmaceutically acceptable salt thereof as a stabilizing agent. Said amino acid may be in the L-form, the D-form or the DL-form. The amino acid is preferably arginine and/or methionine, and more preferably L-arginine and L-methionine.

In the preparation of the present invention, the amount of the amino acid is 0.001 to 1000 parts by weight, preferably 0.01 to 10 parts by weight, and more preferably 0.1 to 5 parts by weight, relative to 1 part by weight of the peptide used.

The preparation of the present invention may comprise, but not limited to, a cyclic oligosaccharide such as cyclodextrin (CD). The cyclodextrin used may be, for example, natural cyclodextrin such as α-cyclodextrin (α-CD), β-cyclodextrin (β-CD) and γ-cyclodextrin (γ-CD), and a cyclodextrin derivative such as hydroxyethyl-β-cyclodextrin (HE-β-CD), hydroxypropyl-p-cyclodextrin (HP-β-CD), methyl-β-cyclodextrin (M-β-CD) and sulfobutyl ether-β-cyclodextrin (SBE-β-CD), or a pharmaceutically acceptable salt thereof, depending on the peptide used. Preferably, the cyclodextrin is β-CD, HP-β-CD, γ-CD, or a pharmaceutically acceptable salt thereof. More preferably, the cyclodextrin is HP-β-CD or a pharmaceutically acceptable salt thereof.

In the preparation of the present invention, the amount of the cyclodextrin is 0.1 to 100 parts by weight, preferably 0.3 to 10 parts by weight, and more preferably 0.5 to 5.0 parts by weight, relative to 1 part by weight of the peptide used.

The preparation of the present invention may be used as a preparation for oral administration such as solid preparation for internal use and solution for internal use and as a preparation for parenteral administration such as an injection, an external preparation, a suppository, an inhalant and a preparation for nasal administration. The solid preparation for internal use may be, for example, a tablet, a pill, a capsule, a powder and a granule. The capsule includes a hard capsule and a soft capsule. Also, the tablet may be, for example, a sublingual tablet, an oral adhesive tablet and an orally rapid-disintegrating tablet.

When the preparation of the present invention is used as an injection, the cancer antigen peptide which is an active ingredient and/or any other ingredients may be provided as a lyophilized preparation. In addition, when the lyophilized preparation comprises a plurality of cancer antigen peptides, the cancer antigen peptides may be lyophilized separately or together.

The preparation of the present invention may be used as a pharmaceutical composition such as CTL inducer and cancer vaccine in cell-mediated immunity therapy for cancer by preparing each compound or each salt thereof in a suitable form.

The peptides disclosed herein, the compounds of formulae (1) - (4), and a pharmaceutically acceptable salt thereof, and a combination thereof may be used in combination with a non-pharmacological therapy. Examples of the non-pharmacological therapy include surgery, radiation therapy, gene therapy, hyperthermia, cryotherapy and laser

burning therapy. Two or more of the non-pharmacological therapies may be used in combination. For example, the peptides, compounds, or a pharmaceutically acceptable salt thereof, or a combination thereof may be used before or after the non-pharmacological therapy such as surgery or before or after a combination therapy of two or three types of the non-pharmacological therapies

The cancer vaccine prepared using the preparation of the present invention may be used as an agent for the treatment or prophylaxis (recurrence prevention) of cancer expressing WT1 genes or cancer with the increased expression levels of WT1 genes. Examples of the cancer include blood cancer such as leukemia, myelodysplastic syndrome, multiple myeloma and malignant lymphoma, and solid cancer such as gastric cancer, colon cancer, lung cancer, breast cancer, germ cell cancer, lung cancer, skin cancer, bladder cancer, prostate cancer, uterine cancer, cervical cancer, ovarian cancer and brain tumor.

The dose of the compound in the preparation of the present invention may be appropriately varied depending on various factors such as diseases for treatment, patient's age and body weight. The dose is usually 0.0001 mg to 1000 mg, preferably 0.001 mg to 1000 mg, and more preferably 0.1 mg to 20 mg.

The preparation of the present invention can stably retain the cancer antigen peptide which is an active ingredient. Hence, the preparation may be used in various administration routes. Specific examples of the routes include oral, nasal, transpulmonary, transdermal, intradermal, subcutaneous, intramuscular, intravenous and intraperitoneal administration. Cancer vaccines for desired purpose may be prepared by the methods described above. In general, parenteral administration is known as the preferred administration route for the immune activation by cancer vaccines. Examples of the parenteral administration include intraperitoneal administration, subcutaneous administration, intradermal administration, intramuscular administration, intravenous administration, nasal administration and transdermal administration. Preferred examples thereof include injectable administration such as intraperitoneal administration, intradermal administration, subcutaneous administration, intramuscular administration, intravenous administration and transdermal administration. More preferred examples thereof include intradermal administration and subcutaneous administration which effectively induce CTLs. The frequency and interval of administration may be appropriately varied depending on diseases for treatment or prophylaxis and individual differences of patients. The frequency and interval of administration are usually multiple times a day, and preferably once every few days or months.

A method of treating or preventing cancer may be provided by administering such preparation comprising the compound of the present invention as an active ingredient to a patient who is WT1 positive.

In the lyophilization process used herein, a killer peptide, a helper peptide, HP-β-CD, and methionine, an organic acid and other ingredients which may optionally be added may be dissolved in purified water and lyophilized. Also, an adjuvant may be dissolved in the prepared solution and lyophilized. The lyophilization process relates to a method of lyophilizing a solution filled into a vial for each formulation under aseptic conditions at low temperature, evaporating solvents contained in a vacuum lyophilizer used in conventional pharmaceutical preparations, and giving lyophilized cakes.

EXAMPLES

Hereinafter, the present invention is specifically explained by Reference Examples and Examples, but is not limited thereto.

In the following Reference Examples and Examples, the compound of the following formula (3):

CRMFPNAPYL
|  (3)
CYTWNQMNL

was used as a "killer peptide" and the peptide consisting of the acid amino sequence: WAPVLDFAPPGASAYGSL (SEQ ID NO: 18) was used as a "helper peptide".

The compound of formula (3) as described above was prepared according to the method described in the patent document (WO 2014/157692). Also, the peptide consisting of the amino acid sequence: WAPVLDFAPPGASAYGSL (SEQ ID NO: 18) was prepared according to the method described in the patent document (WO 2010/123065).

1. Formulation and reconstitution time of lyophilized preparation

<Starting material or reagent used>

| Additive | Product Name | Maker |
|---|---|---|
| D-Mannitol | D(-)-Mannitol EMPROVE api | MERCK |
| Trehalose hydrate | Trehalose SG | HAYASHIBARA |
| L-Methionine | L-Methionine | KYOWA HAKKO BIO |
| Tartaric acid | L-(+)-tartaric acid | MERCK |
| Succinic acid | Succinic acid | NACALAI TESQUE |
| HP-β-Cyclodextrin | Cavitron W7 HP5 PHARMA | ASHLAND |
| α-Cyclodextrin | Cavamax W6 Pharma | ASHLAND |
| γ-Cyclodextrin | Cavamax W8 Pharma | ASHLAND |
| L-Arginine | L-Arginine | NACALAI TESQUE |
| Hydrochloric acid | 1 mol/l Hydrochloric acid | NACALAI TESQUE |

<REFERENCE EXAMPLES>

(1) Preparation of lyophilized preparation

Peptides and other ingredients were dissolved in water for injection so that the amounts (concentrations) thereof shown in the table below were achieved, and a pH adjusting agent (1 mol/L hydrochloric acid) was added to adjust the pH of each solution to 2-3. The prepared solutions were filtrated through a 0.2 μm sterile filter, and then the filtrated solutions were filled into glass vials in a predetermined amount (2 to 2.4 mL) and were lyophilized with a lyophilizer. The lyophilization process was performed under the conditions that comprise freezing the resulting solution at around -40°C, increasing the temperature in the lyophilizer to around -20°C simultaneously with reducing the pressure therein to vacuum and drying for about 20 hours, and then increasing the temperature in the lyophilizer to around 30°C and drying for about 12 hours.

| Reference Examples | 1 | 2 |
|---|---|---|
| Killer peptide | | 10 |
| Helper peptide | 7.5 | |
| D-Mannitol | | 5 |
| Trehalose | 15 | 5 |
| L-Methionine | | 7.5 |
| Tartaric acid | 1.5 | |
| HP-β-Cyclodextrin | | |
| Filled amount (mL) | 2.4 | 2 |
| Reconstitution amount (WFI) (mL) | 1.2 | 1 |
| Reconstitution Time (Mean) | 2m35s | 23s |

(2) Result

For the preparations each comprising the killer peptide and the helper peptide, the reconstitution time when cyclodextrin is not added was less than 3 minutes. The results were relatively good.

On the other hand, for the preparation comprising a mixture of the killer peptide and the helper peptide, it was shown that the reconstitution time when cyclodextrin is not added was 10 minutes or more (see, the following Example 1).

According to the results, it is assumed that the reason why it takes time to reconstitute the preparation comprising a mixture of the killer peptide and the helper peptide like Example 1 is not that the solubility of the killer peptide or the helper peptide is low but that the killer peptide and the helper peptide aggregate in the constituted solution with each other.

<EXAMPLES>

(1) Preparation of lyophilized preparation

Peptides and other ingredients were dissolved in water for injection so that the amounts (concentrations) thereof shown in each table of Formulations (a) - (g) below were achieved, and a pH adjusting agent (1 mol/L hydrochloric acid) was added to adjust the pH of each solution to 2-3. The prepared solutions were filtrated through a 0.2 μm sterile filter, and then the filtrated solutions were filled into glass vials in a predetermined amount (1.2 to 4 mL) and were lyophilized with a lyophilizer. The lyophilization process was performed under the conditions that comprise freezing the resulting solution at around -40°C, increasing the temperature in the lyophilizer to around -20°C simultaneously with reducing the pressure therein to vacuum and drying for about 20 hours, and then increasing the temperature in the lyophilizer to around 30°C and drying for about 12 hours.

(2) Measurement and Assessment of reconstitution time of lyophilized preparation

To each lyophilized preparation was added water for injection in a predetermined amount (1 mL or 0.6 mL) to measure the dissolution time of the preparation required when reconstituted in water.

Formulation a

(a-1) Formulation of lyophilized preparation (mg)

| Examples | 1 | 2 | 3 | 4 | 5 | 6 | 7 | 8 |
|---|---|---|---|---|---|---|---|---|
| Killer peptide | 5 | 5 | 5 | 5 | 5 | 5 | 5 | 5 |
| Helper peptide | 3.75 | 3.75 | 3.75 | 3.75 | 3.75 | 3.75 | 3.75 | 3.75 |
| D-Mannitol | 2.5 | 2.5 | 2.5 | 2.5 | 2.5 | 2.5 | 2.5 | 2.5 |
| Trehalose | 10 | 10 | 10 | 10 | 10 | 10 | 10 | 10 |
| L-Methionine | 3.75 | 3.75 | 3.75 | 3.75 | 3.75 | 3.75 | 3.75 | 3.75 |
| Tartaric acid | 0.75 | | | | | | | |
| Succinic acid | | 1.5 | 1.5 | 1.5 | 1.5 | 1.5 | 0.6 | 0.6 |
| HP-β-Cyclodextrin | | | | | 5 | | | 5 |
| α-Cyclodextrin | | | | 5 | | | | |
| γ-Cyclodextrin | | | | | | 5 | | |
| L-Arginine | | | 2.5 | | | | | |
| Filled amount (mL) | 4 | 4 | 4 | 4 | 4 | 4 | 4 | 4 |
| Reconstitution amount (WFI) (mL) | 1 | 1 | 1 | 1 | 1 | 1 | 1 | 1 |

(a-2) Assessment of reconstitutibility of lyophilized preparation

| Examples | | 1 | 2 | 3 | 4 | 5 | 6 | 7 | 8 |
|---|---|---|---|---|---|---|---|---|---|
| Reconstitution Time | First | 10m 08s | 3m 10s | 6m 20s | 4m 15s | 2m 20s | 2m 50s | 6m 55s | 2m 17s |
| | Second | 9m 58s | 3m 00s | | | 2m 40s | | | |
| | Third | 12m 50s | | | | | | | |
| | Mean | 10m 59s | 3m 05s | 6m 20s | 4m 15s | 2m 30s | 2m 50s | 6m 55s | 2m 17s |

(a-3) Result:

The reconstitution time of the preparation free of succinic acid was 10 minutes or more. On the other hand, the reconstitution time was greatly shortened by the addition of succinic acid. Among the lyophilized preparations comprising succinic acid, the preparations comprising HP-β-cyclodextrin or γ-cyclodextrin further shortened the reconstitution time.

Hence, the results suggested that the addition of succinic acid and HP-β-cyclodextrin or γ-cyclodextrin as cyclodextrin for the killer peptide and the helper peptide of the above examples was effective in shortening the reconstitution time.

Formulation b

(b-1) Formulation of lyophilized preparation (mg)

| Examples | 9 | 10 | 11 | 12 | 13 | 14 |
|---|---|---|---|---|---|---|
| Killer peptide | 5 | 5 | 5 | 5 | 5 | 5 |
| Helper peptide | 3.75 | 3.75 | 3.75 | 3.75 | 3.75 | 3.75 |
| D-Mannitol | 2.5 | 2.5 | 6.25 | 6.25 | 10 | 10 |
| Trehalose | 10 | 10 | 6.25 | 6.25 | 2.5 | 2.5 |
| L-Methionine | 3.75 | 3.75 | 3.75 | 3.75 | 3.75 | 3.75 |
| Tartaric acid | | | | | | |
| Succinic acid | 0.3 | 0.15 | 0.3 | 0.15 | 0.3 | 0.15 |
| HP-β-Cyclodextrin | 5 | 5 | 5 | 5 | 5 | 5 |
| α-Cyclodextrin | | | | | | |
| γ-Cyclodextrin | | | | | | |
| L-Arginine | | | | | | |
| Filled amount (mL) | 4 | 4 | 4 | 4 | 4 | 4 |
| Reconstitution amount (WFI) (mL) | 1 | 1 | 1 | 1 | 1 | 1 |

(b-2) Assessment of reconstitutibility of lyophilized preparation

| Examples | | 9 | 10 | 11 | 12 | 13 | 14 |
|---|---|---|---|---|---|---|---|
| Reconstitution Time | First | 1m 20s | 56s | 53s | 1m 26s | 1m 00s | 1m 06s |
| | Second | 1m 00s | 1m 20s | 1m 50s | 1m 00s | 55s | 45s |
| | Third | | | | | | |
| | Mean | 1m 10s | 1m 08s | 1m 22s | 1m 13s | 58s | 56s |

(b-3) Result:

In the lyophilized preparations comprising succinic acid and HP-β-cyclodextrin, no significant difference was observed in the reconstitution time with respect to the change in the context of D-mannitol, trehalose or L-methionine.

Hence, the results suggested that in the above preparations comprising succinic acid and HP-β-cyclodextrin, the reconstitution time was not affected by the context of D-mannitol, trehalose or L-methionine.

Formulation c

(c-1) Formulation of lyophilized preparation (mg)

| Examples | 15 | 16 | 17 | 18 | 19 | 20 | 21 |
|---|---|---|---|---|---|---|---|
| Killer peptide | 5 | 5 | 5 | 5 | 5 | 5 | 5 |
| Helper peptide | 3.75 | 3.75 | 3.75 | 3.75 | 3.75 | 3.75 | 3.75 |
| D-Mannitol | 2.5 | 2.5 | 2.5 | 2.5 | 2.5 | 2.5 | 2.5 |
| Trehalose | 10 | 10 | 10 | 10 | 10 | 10 | 10 |
| L-Methionine | 3.75 | 3.75 | 3.75 | 3.75 | 3.75 | 3.75 | 3.75 |
| Tartaric acid | | | | | | 0.75 | |
| Succinic acid | 0.3 | 0.15 | 0.15 | 0.15 | 0.3 | | |
| HP-β-Cyclodextrin | 2.5 | 6.25 | 7.5 | 8.75 | 10 | 5 | 5 |
| α-Cyclodextrin | | | | | | | |
| γ-Cyclodextrin | | | | | | | |
| L-Arginine | | | | | | | |
| Filled amount (mL) | 4 | 4 | 4 | 4 | 4 | 4 | 4 |
| Reconstitution amount (WFI) (mL) | 1 | 1 | 1 | 1 | 1 | 1 | 1 |

(c-2) Assessment of reconstitutibility of lyophilized preparation

| Examples | | 15 | 16 | 17 | 18 | 19 | 20 | 21 |
|---|---|---|---|---|---|---|---|---|
| Reconstitution Time | First | 3m 17s | 1m 00s | 34s | 20s | 30s | 1m 18s | 1m 18s |
| | Second | | 59s | 40s | 40s | | | |
| | Third | | | | | | | |
| | Mean | 3m 17s | 1m 00s | 37s | 30s | 30s | 1m 18s | 1m 18s |

(c-3) Result:

When comparing the reconstitution times between the contents of HP-β-cyclodextrin, it was observed that the reconstitution time of the preparation wherein the content of HP-β-cyclodextrin is 6.25 m/L was greatly reduced as compared to that of the preparation wherein the content of HP-β-cyclodextrin is 2.5 m/L. In the preparations wherein the amount of HP-β-cyclodextrin is 7.5 m/L or more, the reconstitution time was remarkably reduced, whereas vigorous bubbling was observed when the preparations were reconstituted. In addition, no effect on reconstitutibility of the lyophilized preparations was observed with or without of tartaric acid.

Hence, the results suggested that the lyophilized preparations comprising HP-β-cyclodextrin at higher concentration were more effective in shortening the reconstitution time. Whereas, when the concentration exceeded a certain level, bubbling was observed when the preparations were reconstituted. As a result, it was suggested that such lyophilized preparations were not preferable in terms of the preparation process. In addition, it was suggested that in the preparations comprising HP-β-cyclodextrin, the addition of succinic acid was effective in shortening the reconstitution time.

Formulation d

(d-1) Formulation of lyophilized preparation (mg)

| Examples | 22 | 23 | 24 | 25 | 26 | 27 | 28 |
|---|---|---|---|---|---|---|---|
| Killer peptide | 10 | 10 | 10 | 10 | 10 | 10 | 10 |
| Helper peptide | 7.5 | 7.5 | 7.5 | 7.5 | 7.5 | 7.5 | 7.5 |
| D-Mannitol | 5 | 5 | 5 | 5 | 5 | 5 | 5 |
| Trehalose | 20 | 20 | 20 | 20 | 20 | 20 | 20 |
| L-Methionine | 7.5 | 7.5 | 7.5 | 7.5 | 7.5 | 7.5 | 7.5 |
| Tartaric acid | | | | | | 1.5 | 1.5 |
| Succinic acid | 3 | 1.2 | 0.6 | 0.3 | 0.3 | | |
| HP-β-Cyclodextrin | 10 | 10 | 10 | 10 | 20 | 10 | 20 |
| α-Cyclodextrin | | | | | | | |
| γ-Cyclodextrin | | | | | | | |
| L-Arginine | | | | | | | |
| Filled amount (mL) | 2 | 2 | 2 | 2 | 2 | 2 | 2 |
| Reconstitution amount (WFI) (mL) | 1 | 1 | 1 | 1 | 1 | 1 | 1 |

(d-2) Assessment of reconstitutibility of lyophilized preparation

| Examples | | 22 | 23 | 24 | 25 | 26 | 27 | 28 |
|---|---|---|---|---|---|---|---|---|
| Reconstitution Time | First | 1m 10s | 1m 54s | 1m 13s | 1m 28s | 30s | 1m 32s | 30s |
| | Second | | | | | | | |
| | Third | | | | | | | |
| | Mean | 1m 10s | 1m 54s | 1m 13s | 1m 28s | 30s | 1m 32s | 30s |

(d-3) Result:

In the lyophilized preparations comprising the peptides at high concentrations, no difference in the reconstitution time was observed between the concentrations of succinic acid. It was observed that the high concentration preparations (Examples 22-25) shortened the reconstitution time as compared to Example 5 or 8. Also, it was observed that when comparing the contents of HP-β-cyclodextrin in the preparations comprising succinic acid or tartaric acid, the preparations comprising HP-β-cyclodextrin at high concentration (Examples 26 and 28) shortened the reconstitution time. Simultaneously, bubbling was observed when the preparations were reconstituted.

Hence, the results suggested that the reconstitution time of the preparations comprising the peptides at high concentrations did not depend on the concentration of succinic acid, and that the preparations comprising HP-β-cyclodextrin at high concentration were not preferable in terms of the preparation process because bubbling was observed in the preparations.

Formulation e

(e-1) Formulation of lyophilized preparation (mg)

| Examples | 29 | 30 | 31 | 32 | 33 |
|---|---|---|---|---|---|
| Killer peptide | 10 | 10 | 10 | 10 | 10 |
| Helper peptide | 7.5 | 7.5 | 7.5 | 7.5 | 7.5 |
| D-Mannitol | 5 | 5 | 5 | 20 | 25 |
| Trehalose | 20 | 20 | 20 | 5 | |
| L-Methionine | 7.5 | 7.5 | 7.5 | 7.5 | 7.5 |
| Tartaric acid | 1.5 | 1.5 | 1.5 | 1.5 | 1.5 |
| Succinic acid | | | | | |
| HP-β-Cyclodextrin | 20 | 10 | 5 | 10 | 10 |
| α-Cyclodextrin | | | | | |
| γ-Cyclodextrin | | | | | |
| L-Arginine | | | | | |
| Filled amount (mL) | 2 | 2 | 2 | 2 | 2 |
| Reconstitution amount (WFI) (mL) | 1 | 1 | 1 | 1 | 1 |

(e-2) Assessment of reconstitutibility of lyophilized preparation

| Examples | | 29 | 30 | 31 | 32 | 33 |
|---|---|---|---|---|---|---|
| Reconstitution Time | First | 24s | 39s | 8m 49s | 57s | 1m 21s |
| | Second | 21s | 1m 22s | | 56s | 1m 05s |
| | Third | | | | | |
| | Mean | 23s | 1m 01s | 8m 49s | 57s | 1m 13s |

(e-3) Result:

In the lyophilized preparations comprising the peptides at high concentrations, the significant difference in the reconstitution time was observed between the concentrations of HP-β-cyclodextrin. On the other hand, in the preparation comprising HP-β-cyclodextrin at a concentration of 20 m/L (Example 29), bubbling was observed when the preparation was reconstituted.
In the lyophilized preparations comprising the peptides at high concentrations, no difference in the reconstitution time was observed between the concentrations of D-mannitol or trehalose.
Hence, the results suggested that the reconstitution time of the preparations depended on the concentration of HP-β-cyclodextrin, whereas the preparations comprising HP-β-cyclodextrin at high concentration were not preferable in terms of the preparation process because bubbling was observed in the preparations.

Formulation f

(f-1) Formulation of lyophilized preparation (mg)

| Examples | 34 | 35 | 36 | 37 | 38 | 39 | 40 |
|---|---|---|---|---|---|---|---|
| Killer peptide | 10 | 10 | 10 | 10 | 10 | 10 | 10 |
| Helper peptide | 7.5 | 7.5 | 7.5 | 7.5 | 7.5 | 7.5 | 7.5 |
| D-Mannitol | 5 | 5 | 5 | 5 | 5 | 5 | 5 |
| Trehalose | 20 | 20 | 20 | 20 | 20 | 20 | 20 |
| L-methionine | 7.5 | 5 | 2.5 | | | 2.5 | |
| Tartaric acid | 1.5 | 1.5 | 1.5 | 1.5 | 1.5 | 1.5 | 1.5 |
| Succinic acid | | | | | | | |
| HP-β-cyclodextrin | 10 | 10 | 10 | 10 | 10 | 10 | 20 |
| α-cyclodextrin | | | | | | | |
| γ-cyclodextrin | | | | | | | |
| L-arginine | | | | | | | |
| Filled amount (mL) | 2 | 2 | 2 | 2 | 1.2 | 1.2 | 1.2 |
| Reconstitution amount (WFI) (mL) | 1 | 1 | 1 | 1 | 0.6 | 0.6 | 0.6 |

(f-2) Assessment of reconstitutibility of lyophilized preparation

| Examples | | 34 | 35 | 36 | 37 | 38 | 39 | 40 |
|---|---|---|---|---|---|---|---|---|
| Reconstitution Time | First | 1m 35s | 1m 07s | 34s | 40s | 40s | 20s | 20s |
| | Second | | | | | | | |
| | Third | | | | | | | |
| | Mean | 1m 35s | 1m 07s | 34s | 40s | 40s | 20s | 20s |

(f-3) Result:

In the lyophilized preparations comprising the peptides at high concentrations, the reconstitution time was shortened when methionine is contained at low concentration or is not contained. On the other hand, in the preparation comprising HP-β-cyclodextrin at a concentration of 20 m/L (Example 44), bubbling was observed when the preparation was reconstituted.

Hence, the results suggested that there was a negative correlation between the reconstitution time and the concentration of methionine, and reducing the methionine concentration was effective in shortening the reconstitution time.

Formulation g

(g-1) Formulation of lyophilized preparation (mg)

| Examples | 41 | 42 | 43 | 44 | 45 |
|---|---|---|---|---|---|
| Killer peptide | 5 | 5 | 5 | 5 | 5 |
| Helper peptide | 3.75 | 3.75 | 3.75 | 3.75 | 3.75 |
| D-Mannitol | 2.5 | 2.5 | 12.5 | 2.5 | 2.5 |
| Trehalose | | 10 | | 10 | 10 |
| L-Methionine | | 1.25 | | | 1.25 |
| Tartaric acid | 0.75 | 0.75 | 0.75 | 0.75 | 0.75 |
| Succinic acid | | | | | |
| HP-β-cyclodextrin | 5 | 5 | 5 | 5 | 5 |
| α-Cyclodextrin | | | | | |
| γ-Cyclodextrin | | | | | |
| L-Arginine | | | | | |
| Filled amount (mL) | 4 | 4 | 4 | 4 | 4 |
| Reconstitution amount (WFI) (mL) | 1 | 1 | 1 | 1 | 1 |

(g-2) Assessment of reconstitutibility of lyophilized preparation

| Examples | | 41 | 42 | 43 | 44 | 45 |
|---|---|---|---|---|---|---|
| Reconstitution Time | First | 45s | 45s | 47s | 38s | 35s |
| | Second | | | | | |
| | Third | | | | | |
| | Mean | 45s | 45s | 47s | 38s | 35s |

(g-3) Result:

The reconstitution time was not affected by the ratio of excipients contained such as D-mannitol, trehalose and L-methionine. On the other hand, the difference in the reconstitution time was not observed when L-methionine was not added or was added in low concentration (1.25 m/L).

Hence, the results suggested that the reconstitution time was not affected by the concentration of D-mannnitol and the presence or absence of trehalose or L-methionine.

(h-1) Preparation of each liquid preparation

One or more peptides consisting of the amino acid sequence selected from SEQ ID NOs: 2-10 and 21-33 or a compound of formula (2) or (4) or/and peptides consisting of the amino acid sequence selected from SEQ ID NOs: 11-20 and 34-40 used instead of the killer peptide and the helper peptide shown in each table of Formulations (a) to (g) described above and other ingredients are dissolved in water for injection so that the amounts (concentrations) thereof shown in each table are achieved, and a pH adjusting agent (1 mol/L hydrochloric acid) is added to adjust the pH of each solution to 2-3. The prepared solutions are filtrated through a 0.2 μm sterile filter, and then the filtrated solutions are filled into glass vials in a predetermined amount (1.2 to 4 mL) and are lyophilized with a lyophilizer. The lyophilization process is performed under the conditions that comprise freezing the resulting solution at around - 40°C, increasing the temperature in the lyophilizer to around -20°C simultaneously with reducing the pressure therein to vacuum and drying for about 20 hours, and then increasing the temperature in the lyophilizer to around 30°C and drying for about 12 hours.

(h-2) Result:

The results of the above examples show that (a) the addition of succinic acid and the addition of HP-β-cyclodextrin or γ-cyclodextrin for the peptides listed in the examples are effective in shortening the reconstitution time; (b) in the preparations comprising succinic acid and HP-β-cyclodextrin, the reconstitution time is not affected by the context of D-mannitol, trehalose or L-methionine; (c) the lyophilized preparations comprising HP-β-cyclodextrin at higher concentration are more effective in shortening the reconstitution time, the lyophilized preparations comprising HP-β-cyclodextrin at a concentration above a certain level are not preferable in terms of the preparation process because bubbling is observed when the preparations are reconstituted, and in the preparations comprising HP-β-cyclodextrin, the addition of succinic acid is effective in shortening the reconstitution time; (d) the reconstitution time of the preparations comprising the peptides at high concentrations does not depend on the concentration of succinic acid, and the preparations comprising HP-β-cyclodextrin at high concentration are not preferable in terms of the preparation process because bubbling is observed in the preparations; (e) the reconstitution time of the preparations depends on the concentration of HP-β-cyclodextrin, whereas the preparations comprising HP-β-cyclodextrin at high concentration are not preferable in terms of the preparation process because bubbling is observed in the preparations; (f) there is a negative correlation between the reconstitution time and the concentration of methionine, and reducing the methionine concentration is effective effective in shortening the reconstitution time; and (g) in the preparations comprising succinic acid and HP-β-cyclodextrin, the reconstitution time is not affected by the context of D-mannitol, trehalose or L-methionine.

In view of the foregoing, it is found that the preparations comprising one or more peptides consisting of the amino acid sequence selected from SEQ ID NOs: 2-10 and 21-33 or a compound of formula (2) or (4) or/and peptides consisting of the amino acid sequence selected from SEQ ID NOs: 11-20 and 34-40 and cyclodextrin can also shorten the reconstitution time. In addition, the preparations may comprise other ingredient(s), for example, an organic acid such as succinic acid, an excipient such as D-mannitol, trehalose and L-methionine.

2. Effect for inhibiting insoluble product in preparation process

The effect for inhibiting an insoluble product in the preparation process was assessed for the following preparations.

<Starting material or reagent used>

| Additive | Product Name | Maker |
|---|---|---|
| D-Mannitol | D(-)-Mannitol EMPROVE api | MERCK |

(continued)

| Additive | Product Name | Maker |
|---|---|---|
| Trehalose hydrate | $\alpha,\alpha$-Trehalose Dihydrate | Pfanstiehl |
| L-Methionine | L-Methionine | Sigma Aldrich |
| Tartaric acid | NATURL L(+) tartaric acid | Distillerie Mazzari |
| HP-$\beta$-Cyclodextrin | Cavitron W7 HP5 PHARMA | ASHLAND |
| L-Arginine | L-Arginine | NACALAI TESQUE |
| Sucrose | Sucrose (Saccharose) | NACALAI TESQUE |
| Sodium chloride | Sodium chloride | NACALAI TESQUE |
| Sodium acetate | Sodium acetate | NACALAI TESQUE |
| Polysorbate 80 | Tween Ph Eur,NF,JP | MERCK |
| CMC-Na | Aqualon | ASHLAND |
| PEG4000 | PEG#4000 | NOF |
| Polysorbate 20 | NIKKOL TL-10 | NIKKO CHEMICALS |
| Hydrochloric acid | 1 mol/l Hydrochloric acid | NACALAI TESQUE |

(1-1) Preparation of each liquid formulation

Peptides and other ingredients were dissolved in water for injection so that the amounts (concentrations) thereof shown in each formulation table below were achieved, and a pH adjusting agent (1 mol/L hydrochloric acid) was added to adjust the pH of each solution to 2-3. The prepared solutions were stirred at room temperature overnight (for 14 to 16 hours) to assess the property of each solution.

| Examples | 46 | 47 | 48 | 49 | 50 | 51 | 52 | 53 |
|---|---|---|---|---|---|---|---|---|
| Killer peptide | 10 | 10 | 10 | 10 | 10 | 10 | 10 | 5 |
| Helper peptide | 7.5 | 7.5 | 7.5 | 7.5 | 7.5 | 7.5 | 7.5 | 3.75 |
| D-Mannitol | 5 | 5 | 5 | 5 | 5 | 5 | 5 | 2.5 |
| Trehalose | 20 | 20 | 20 | 20 | 20 | 20 | 20 | 10 |
| L-Methionine | 7.5 | | 7.5 | 7.5 | 7.5 | 7.5 | 7.5 | 3.75 |
| Tartaric acid | 1.5 | 1.5 | 1.5 | 1.5 | 1.5 | 1.5 | 1.5 | 0.75 |
| HP-$\beta$-Cyclodextrin | 30 | 10 | 10 | 10 | 10 | 10 | 10 | 5 |
| L-Arginine | | | 5 | | | | | |
| Sucrose | | | | 5 | | | | |
| Sodium chloride | | | | | 5 | | | |
| Sodium acetate | | | | | | 5 | | |
| Polysorbate 80 | | | | | | | 5 | |

| Examples | 54 | 55 | 56 | 57 | 58 | 59 | 60 |
|---|---|---|---|---|---|---|---|
| Killer peptide | 10 | 10 | 10 | 5 | 10 | 10 | 10 |
| Helper peptide | 7.5 | 7.5 | 7.5 | 3.75 | 7.5 | 7.5 | 7.5 |
| D-Mannitol | 5 | | 5 | 5 | 5 | 5 | 5 |

(continued)

| Examples | 54 | 55 | 56 | 57 | 58 | 59 | 60 |
|---|---|---|---|---|---|---|---|
| Trehalose | 20 | | 20 | 20 | 20 | 20 | 20 |
| L-methionine | | | | | | | |
| Tartaric acid | 1.5 | 1.5 | | 1.5 | 1.5 | 1.5 | 1.5 |
| HP-β-Cyclodextrin | 10 | 10 | 10 | 10 | 10 | 10 | 10 |
| CMC-Na | | | | | 5 | | |
| PEG4000 | | | | | | 5 | |
| Polysorbate 20 | | | | | | | 5 |

(1-2) Assessment of property of each liquid preparation after standing

The liquid preparations prepared with the compositions shown in each formulation table described above were allowed to stand overnight (for 14 or 16 hours) to assess whether insoluble products were contained in the solutions. In addition, in the preparations of Examples 57 and 59, it was confirmed whether insoluble products were contained in the solutions after 23 hours.

| Examples | 46 | 47 | 48 | 49 | 50 | 51 | 52 | 53 |
|---|---|---|---|---|---|---|---|---|
| After 16 hr | slight insoluble products | clear | cloudy | cloudy | cloudy | cloudy | cloudy | clear |

| Examples | 54 | 55 | 56 |
|---|---|---|---|
| After 14 hr | Particle generation Aggregates | Particle generation Aggregates | Particle generation |

| Examples | 57 | 58 | 59 | 60 |
|---|---|---|---|---|
| After 14 hr | clear | not be prepared | clear | Particle generation |
| After 23 hr | clear | | Particle generation Aggregates | |

(1-3) Result

Regarding the preparations of Examples 46-53, the preparations comprising HP-β-cyclodextrin at high concentration could not inhibit the generation of insoluble products, but the preparation of Example 47 free of methionine could inhibit the generation of insoluble products.

The generation of insoluble products could not be inhibited by the addition of an aggregation inhibitor and a solubilizing agent, but could be inhibited by reducing the bulk concentration to 1/2.

Hence, the results suggested that no methionine or the reduced bulk concentration was effective in inhibiting the generation of insoluble products.

In Example 58, a solution could not be prepared because CMC-Na was not dissolved.

All of the preparations other than Example 58 were clear immediately after being prepared. However, in the preparations of Examples 54, 55, 56 and 60, particles were generated and/or aggregates were confirmed after 14 hours. In the preparation comprising PEG4000, particles were generated and aggregates were confirmed after 23 hours. On the other hand, the preparation of Example 57 adjusted the bulk concentration to 1/2 maintained the clear property even after 23 hours.

Hence, the results suggested that the generation of insoluble products in the preparation process were inhibited under the conditions with no methionine or low concentration of methionine, and also that the generation of insoluble products could be inhibited for a longer time when the bulk concentration is adjusted to low concentration.

(2-1) Preparation of each liquid preparation

One or more peptides consisting of the amino acid sequence selected from SEQ ID NOs: 2-10 and 21-33 or a compound of formula (2) or (4) or/and peptides consisting of the amino acid sequence selected from SEQ ID NOs: 11-20 and 34-40 used instead of the killer peptide and the helper peptide shown in each formulation described above and other ingredients are dissolved in water for injection so that the amounts (concentrations) thereof shown in each formulation are achieved, and a pH adjusting agent (1 mol/L hydrochloric acid) is added to adjust the pH of each solution to 2-3. The solutions are stirred at room temperature overnight (for 14 to 16 hours) to assess the property of each solution.

(2-2) Result

The results of the above examples show that no methionine or the reduced bulk concentration is effective in inhibiting the generation of insoluble products, that the generation of insoluble products in the preparation process is inhibited under the conditions with no methionine or low concentration of methionine, and also that the generation of insoluble products is inhibited for a longer time when the bulk concentration is adjusted to low concentration.

In view of the foregoing, it is found that the preparations comprising one or more peptides consisting of the amino acid sequence selected from SEQ ID NOs: 2-10 and 21-33 or a compound of formula (2) or (4) or/and peptides consisting of the amino acid sequence selected from SEQ ID NOs: 11-20 and 34-40 and cyclodextrin can also produce the effect for inhibiting the generation of insoluble products, and that the preparations can inhibit the generation of insoluble products for a long time when the bulk concentration is adjusted at a certain level.

3. Confirmation of stability of lyophilized preparation

<Starting material or reagent used>

| Additive | Product Name | Maker |
|---|---|---|
| D-Mannitol | D(-)-Mannitol EMPROVE api | MERCK |
| Trehalose hydrate | α,α-Trehalose Dihydrate | Pfanstiehl |
| L-Methionine | L-methionine | KYOWA HAKKO BIO |
| Tartaric acid | NATURL L(+) tartaric acid | Distillerie Mazzari |
| HP-β-cyclodextrin | Cavitron W7 HP5 PHARMA | ASHLAND |
| Hydrochloric acid | 1 mol/l Hydrochloric acid | NACALAI TESQUE |

(1-1) Preparation of lyophilized preparation

Peptides and other ingredients were dissolved in water for injection so that the amounts (concentrations) thereof shown in the table below were achieved, and a pH adjusting agent (1 mol/L hydrochloric acid) was added to adjust the pH of each solution to 2-3. The prepared solutions were filtrated through a 0.2 μm sterile filter, and then the filtrated solutions were filled into glass vials in a predetermined amount (4 mL) and were lyophilized with a lyophilizer. The lyophilization process was performed under the conditions that comprise freezing the resulting solution at around -40°C, increasing the temperature in the lyophilizer to around -20°C simultaneously with reducing the pressure therein to vacuum and drying for about 20 hours, and then increasing the temperature in the lyophilizer to around 30°C and drying for about 12 hours.

| Examples | Comparative Example 1 | 61 | 62 | 63 | 64 | 65 | 66 |
|---|---|---|---|---|---|---|---|
| Killer peptide | | 5 | 5 | 10 | 10 | 10 | 10 |
| Helper peptide | 7.5 | 3.7 5 | 3.7 5 | 7.5 | 7.5 | 7.5 | 7.5 |
| D-Mannitol | | 2.5 | 2.5 | 5 | 5 | 5 | 5 |
| Trehalose | 15 | 10 | 10 | 20 | 20 | 20 | 20 |

(continued)

| Examples | Comparative Example 1 | 61 | 62 | 63 | 64 | 65 | 66 |
|---|---|---|---|---|---|---|---|
| L-Methionine | | | 1.2 5 | 7.5 | 5 | 2.5 | |
| Tartaric acid | 1.5 | 0.7 5 | 0.7 5 | 1.5 | 1.5 | 1.5 | 1.5 |
| HP-β-cyclodextrin | | 5 | 5 | 10 | 10 | 10 | 10 |
| Filled amount (mL) | 2.4 | 4 | 4 | 2 | 2 | 2 | 2 |
| Reconstitution amount (WFI) (mL) | 1.2 | 1 | 1 | 1 | 1 | 1 | 1 |
| Reconstitution Time | | - | 38s | 35s | 1m 35s | 1m 07s | 34s |

(1-2) Assessment of stability of lyophilized preparation

Each lyophilized preparation was preserved for 2 weeks and 1 month at 5°C, 25°C or 40°C when reconstituted. The preparations were dissolved in water for injection in a predetermined amount (1 mL or 1.2 mL) to assess the amount of the analog (RRT.0.875). The amount of the analog (RRT.0.875) was measured by a reversed-phase high performance liquid chromatography method (detection wavelength: 220 nm) using C18 reversed-phase column (2.1 mm x 100 mm, 1.7 pm) and pure water, acetonitrile, trifluoroacetic acid as mobile phase. The amount of the analog (RRT.0875) was calculated according to the following formula.

Amount of analog (RRT.0875) (%) = peak area of analog (RRT.0875) / peak area of DSR-131617 x 100

Comparative Example 1 and Examples 61-62 (Low concentration formulation)

| | Comparative Example 1 | | | Example 61 | | | Example 62 | | |
|---|---|---|---|---|---|---|---|---|---|
| | 5°C | 25°C | 40 ° | 5°C | 25°C | 40°C | 5°C | 25°C | 40°C |
| Initial | 0.15 | | | 0.17 | | | 0.15 | | |
| 2W | | | | | | 0.40 | | | 0.27 |
| 1M | | | | 0.18 | 0.26 | 0.60 | 0.16 | 0.18 | 0.32 |
| 3M | 0.17 | 0.20 | | 0.19 | 0.43 | | 0.16 | 0.21 | |
| 6M | 0.18 | 0.21 | | 0.22 | 0.53 | | 0.18 | 0.29 | |

Examples 63-66 (High concentration formulation)

| | Example 63 | | Example 64 | | Example 65 | | Example 66 | |
|---|---|---|---|---|---|---|---|---|
| | 25°C | 40°C | 25°C | 40°C | 25°C | 40°C | 25°C | 40°C |
| Initial | 0.17 | | 0.15 | | 0.15 | | 0.15 | |
| 2W | | 0.19 | | 0.17 | 0.21 | 0.21 | | 0.24 |
| 1M | 0.15 | 0.21 | 0.19 | 0.18 | 0.16 | 0.20 | 0.17 | 0.30 |

(1-3) Result

The preparations comprising methionine at low concentration (1.25 mg/mL or 2.5 mg/mL) inhibited the generation of analogs of the peptides during long-term storage as compared to the preparations free of methionine.

Hence, the results suggested that the addition of methionine involved in the inhibition of the generation of analogs during long-term storage.

(2-1) Preparation of each liquid preparation

One or more peptides consisting of the amino acid sequence selected from SEQ ID NOs: 2-10 and 21-33 or a compound of formula (2) or (4) or/and peptides consisting of the amino acid sequence selected from SEQ ID NOs: 11-20 and 34-40 used instead of the killer peptide and the helper peptide shown in the table described above and other ingredients are dissolved in water for injection so that the amounts (concentrations) thereof shown in the table are achieved, and a pH adjusting agent (1 mol/L hydrochloric acid) is added to adjust the pH of each solution to 2-3. The prepared solutions are filtrated through a 0.2 μm sterile filter, and then the filtrated solutions are filled into glass vials in a predetermined amount (4 mL) and are lyophilized with a lyophilizer. The lyophilization process is performed under the conditions that comprise freezing the resulting solution at around -40°C, increasing the temperature in the lyophilizer to around -20°C simultaneously with reducing the pressure therein to vacuum and drying for about 20 hours, and then increasing the temperature in the lyophilizer to around 30°C and drying for about 12 hours.

(2-2) Result

The results of the above examples show that the addition of methionine involves in the inhibition of the generation of analogs during long-term storage.

In view of the foregoing, the preparations comprising one or more peptides consisting of the amino acid sequence selected from SEQ ID NOs: 2-10 and 21-33 or a compound of formula (2) or (4) or/and peptides consisting of the amino acid sequence selected from SEQ ID NOs: 11-20 and 34-40 and cyclodextrin can inhibit the generation of analogs during long-term storage.

## Claims

1. A lyophilized preparation comprising a WT1 killer peptide, a WT1 helper peptide and cyclodextrin, wherein the WT1 killer peptide comprises a compound selected from:

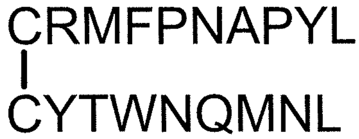

(3)

or

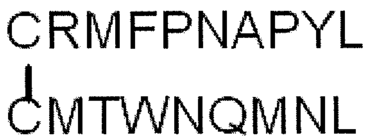

(4)

,

and
the WT1 helper peptide comprises a peptide consisting of the amino acid sequence: WAPVLDFAPPGASAYGSL (SEQ ID NO: 18).

2. The lyophilized preparation according to claim 1, wherein the WT1 killer peptide is a compound of formula (3)

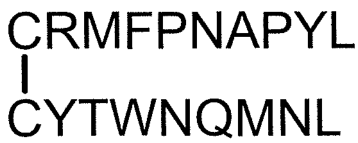

(3)

.

3. The lyophilized preparation according to claim 1 or 2, wherein the amounts of the WT1 helper peptide and cyclodextrin are 0.1 to 2.0 parts by weight and 0.2 to 5.0 parts by weight, respectively, relative to 1 part by weight of the WT1 killer peptide.

4. The lyophilized preparation according to any one of claims 1-3, wherein the amounts of the WT1 helper peptide and cyclodextrin are 0.5 to 1.0 part by weight and 0.5 to 2.0 parts by weight, respectively, relative to 1 part by weight of the WT1 killer peptide.

5. The lyophilized preparation according to any one of claims 1-4, wherein the cyclodextrin is one or more selected from the group consisting of α-cyclodextrin (α-CD), β-cyclodextrin (β-CD), γ-cyclodextrin (γ-CD), hydroxyethyl-β-cyclodextrin (HE-β-CD), hydroxypropyl-β-cyclodextrin (HP-β-CD), methyl-β-cyclodextrin (M-β-CD), sulfobutyl ether-β-cyclodextrin (SBE-β-CD) and a pharmaceutically acceptable salt thereof.

6. The lyophilized preparation according to any one of claims 1-5, wherein the cyclodextrin is one or more selected from the group consisting of α-cyclodextrin (α-CD), γ-cyclodextrin (γ-CD), hydroxypropyl-β-cyclodextrin (HP-β-CD) and a pharmaceutically acceptable salt thereof.

7. The lyophilized preparation according to any one of claims 1-6, wherein the cyclodextrin is hydroxypropyl-β-cyclodextrin (HP-β-CD) or a pharmaceutically acceptable salt thereof.

8. The lyophilized preparation according to any one of claims 1-7, which further comprises arginine or methionine.

**9.** The lyophilized preparation according to claim 8, wherein the amount of arginine or methionine is 0.01 to 1.0 part by weight relative to 1 part by weight of the WT1 helper peptide.

**10.** The lyophilized preparation according to claim 8 or 9, wherein the amount of arginine or methionine is 0.1 to 0.5 part by weight relative to 1 part by weight of the WT1 helper peptide.

**11.** The lyophilized preparation according to any one of claims 8-10, which comprises L-methionine as the methionine.

**12.** The lyophilized preparation according to any one of claims 1-11, which further comprises an organic acid.

**13.** The lyophilized preparation according to claim 12, wherein the amount of the organic acid is 0.01 to 1.0 part by weight relative to 1 part by weight of the WT1 helper peptide.

**14.** The lyophilized preparation according to claim 12 or 13, wherein the amount of the organic acid is 0.05 to 0.5 part by weight relative to 1 part by weight of the WT1 helper peptide.

**15.** The lyophilized preparation according to any one of claims 12-14, wherein the organic acid is tartaric acid and/or succinic acid.

**16.** The lyophilized preparation according to any one of claims 1-15, which further comprises an adjuvant.

**17.** The lyophilized preparation according to claim 16, wherein the amount of the adjuvant is 1 to 500 parts by weight relative to 1 part by weight of the WT1 killer peptide.

**18.** The lyophilized preparation according to claim 16 or 17, wherein the amount of the adjuvant is 5 to 100 parts by weight relative to 1 part by weight of the WT1 killer peptide.

**19.** The lyophilized preparation according to any one of claims 16-18, wherein the adjuvant is one of Freund's adjuvant, Montanide and W/O emulsion.

**20.** The lyophilized preparation according to any one of claims 16 to 19, wherein the adjuvant is Montanide.

**21.** The lyophilized preparation according to any one of claims 1 to 20 produced by dissolving each ingredient in purified water in an amount of 0.5 to 2 mL per 5 mg of the WT1 killer peptide and lyophilizing the solution.

**INTERNATIONAL SEARCH REPORT**

International application No. PCT/JP2019/038223

A. CLASSIFICATION OF SUBJECT MATTER
Int.Cl. A61K39/00(2006.01)i, A61K38/1(2006.01)i, A61K38/16(2006.01)i, A61K47/69(2017.01)i, A61P35/00(2006.01)i, C07K14/82(2006.01)n, C07K19/00(2006.01)n
According to International Patent Classification (IPC) or to both national classification and IPC

B. FIELDS SEARCHED
Minimum documentation searched (classification system followed by classification symbols)
Int.Cl. A61K39/00, A61K38/10, A61K38/16, A61K47/69, A61P35/00, C07K14/82, C07K19/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII), CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| Y | WO 2016/047797 A1 (SUMITOMO DAINIPPON PHARMA CO., LTD.) 31 March 2016, claims, examples, paragraphs [0087], [0089], [0092] & US 2018/0344831 A1, claims, examples, paragraphs [0126], [0129], [0133] & EP 3199175 A1 & CN 106687129 A | 1-21 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 November 2019 (22.11.2019) | 10 December 2019 (10.12.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/038223

C (Continuation). DOCUMENTS CONSIDERED TO BE RELEVANT

| Category* | Citation of document, with indication, where appropriate, of the relevant passages | Relevant to claim No. |
|---|---|---|
| Y | BREWSTER, M. E., et al., "Use of 2-hydroxypropy 1-beta-cyclodextrin as a solubilizing and stabilizing excipient for protein drugs.", Pharm. Res., 1991, vol. 8, no. 6, pp. 792-795, abstract | 1-21 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0006602 A
- WO 0018795 A
- WO 2007063903 A
- WO 2014157692 A
- WO 2010123065 A
- WO 2016047797 A
- WO 03106682 A
- WO 2009072610 A
- WO 0279253 A
- WO 2004026897 A
- WO 2003106682 A
- WO 2007120673 A
- WO 2000006602 A
- WO 2002079253 A
- WO 2004063903 A
- WO 2018101309 A
- WO 2005053618 A
- WO 2007047764 A
- WO 2005045027 A
- WO 2010037395 A
- WO 2000018795 A
- WO 2002028414 A
- WO 2003037060 A
- WO 2004100870 A
- WO 0247474 A
- WO 2006078059 A
- WO 2016186177 A


### Non-patent literature cited in the description


- *Blood,* 2010, vol. 166 (2), 177-179
- *J. Immunol.,* 1994, vol. 152, 3913
- *J. Immunol.,* 1994, vol. 155, 4307
- *Immunogenetics,* 1995, vol. 41, 178
- Peptide Synthesis. Interscience, 1966
- The Proteins. Academic Press Inc, 1976, vol. 2
- Peptide Synthesis. Maruzen Co., Ltd, 1975
- Basic and Experiment of Peptide Synthesis. Maruzen Co., Ltd, 1985
- Development of Pharmaceutical Product subsequent. Peptide Synthesis. Hirokawa Shoten, 1991, vol. 14
- Protective Groups in Organic Synthesis. John Wiley & Sons, Inc, 1990
- *Development of Pharmaceutical Product subsequent,* vol. 14
- Peptide Synthesis. Hirokawa Shoten, 1991
- *Tetrahedron Letters,* vol. 37 (9), 1347-1350
- *Int. J. Cancer,* 2002, vol. 100, 565-570
- *Nat. Med.,* 1998, vol. 4, 321-327
- *Cancer Immunol. Immunother,* 2002, vol. 51, 271
- *Clin. Microbiol. Rev.,* 1994, vol. 7, 277-289
- *Nat Rev Drug Discov,* 2013, vol. 12, 130-146
- *Nikkei Medical Cancer Review,* 2014, vol. 9
- *Nat Rev Immunol.,* 2014, vol. 14, 559-69